# EUROPÄISCHE PATENTANMELDUNG

(11) **EP 4 387 052 A1**
(43) Veröffentlichungstag der Anmeldung: **19.06.2024**
(21) Anmeldenummer: 23212483.4
(22) Anmeldetag: 28.11.2023
(51) Int. Cl.: H02K 1/14, H02K 5/04, H02K 5/128, H02K 15/00, C12M 1/00, C12M 1/26, C12M 1/12, H02K 7/09

(54) **ELEKTROMAGNETISCHER DREHANTRIEB**

(30) Priorität: 12.12.2022 EP 22212875
(71) Anmelder: Levitronix GmbH, 8048 Zürich (CH)
(72) Erfinder: Steinert, Daniel, 8180 Bülach (CH); Hubmann, Emanuel, 8003 Zürich (CH); Schöb, Reto, 8832 Wollerau (CH)
(74) Vertreter: IPS Irsch AG

(57) **Zusammenfassung**

Es wird ein elektromagnetischer Drehantrieb vorgeschlagen mit einer Einmalvorrichtung (100), die für den Einmalgebrauch ausgestaltet ist, und mit einer wiederverwendbaren Vorrichtung (200), die für den Mehrfachgebrauch ausgestaltet ist, wobei die Einmalvorrichtung (100) ein Rotorgehäuse (2) mit einem Rotor (3), einen Verbindungsschlauch (9) für ein Fluid, und mindestens eine weitere Einmalkomponente (10) umfasst, wobei das Rotorgehäuse (2) einen Einlass (21) und einen Auslass (22) aufweist, wobei der Rotor (3) in dem Rotorgehäuse (2) zum Rotieren um eine Solldrehachse vorgesehen ist, welche eine axiale Richtung (A) definiert, wobei der Rotor (3) berührungslos magnetisch antreibbar ist, und wobei der Verbindungsschlauch (9) den Einlass (21) oder den Auslass (22) des Rotorgehäuses (2) mit der weiteren Einmalkomponente (10) verbindet, derart, dass im Betriebszustand das Fluid den Verbindungsschlauch (9) durchströmen kann, wobei die wiederverwendbare Vorrichtung (200) einen Stator (4) umfasst, mit welchem der Rotor (3) im Betriebszustand berührungslos magnetisch zur Rotation um die Solldrehachse antreibbar ist, wobei der Stator (4) eine Mehrzahl von Wicklungen (6) zur Erzeugung eines elektromagnetischen Drehfelds zum Antreiben des Rotors (3) umfasst, und wobei der Stator (4) ein Statorgehäuse (5) mit einer Durchführung (52) für den Verbindungsschlauch (9) aufweist. Der Stator (4) ist öffenbar mit einem ersten Statorteil (48) und einem zweiten Statorteil (49) ausgestaltet, welche relativ zueinander bewegbar sind, wobei in einer Offenstellung des Stators (4) der Verbindungsschlauch (9) in die Durchführung (52) einlegbar ist, und wobei in einer Schliessstellung des Stators (4) die Durchführung (52) den Verbindungsschlauch (9) umschliesst. Ferner wird eine Einmalvorrichtung (100) für einen solchen elektromagnetischen Drehantrieb vorgeschlagen.

## Beschreibung

Die Erfindung betrifft einen elektromagnetischen Drehantrieb gemäss dem Oberbegriff des unabhängigen Patentanspruchs 1. Die Erfindung betrifft ferner eine Einmalvorrichtung für einen solchen elektromagnetischen Drehantrieb.

In der biotechnologischen und in der pharmazeutischen Industrie werden häufig elektromagnetische Drehantriebe eingesetzt, die zum Antreiben von Pumpen, Mischern, Separatoren, Filtervorrichtungen oder ähnlichem dienen. Die Pumpen dienen beispielsweise dazu, Fluide durch einen Kreislauf mit einem Bioreaktor zu fördern. Mit Mischern wird beispielsweise eine kontinuierliche Durchmischung und Zirkulation der Nährflüssigkeit in einem Bioreaktor realisiert. Mit Separatoren, die häufig nach dem Prinzip einer Zentrifuge arbeiten, werden beispielsweise in biotechnologischen Prozessen fliessfähige Substanzen, z.B. Suspensionen in zwei Phasen unterschiedlicher Dichte aufgeteilt. Filtervorrichtungen dienen beispielsweise dazu, um Stoffwechselprodukte von Zellen von einem Fluid zu separieren.

Bioreaktoren werden zur Gewinnung von Substanzen, beispielsweise Proteinen, oder zur Züchtung von Zellen oder anderem biologischen Material eingesetzt. Dabei können Bioreaktoren sowohl in kontinuierlichen Prozessen als auch in Batch Prozessen betrieben werden. Bei diesen Verfahren ist es eine übliche Methode, das Fluid, beispielsweise eine Zellbrühe (cell broth), dem Bioreaktor zu entnehmen, einer Filtervorrichtung zuzuführen, und das Retentat wieder in den Bioreaktor zurückzuführen. Die zu extrahierende Substanz wird dann als Filtrat bzw. als Permeat der Filtervorrichtung entnommen und abgeführt.

Für solche Prozesse werden elektromagnetische Drehantriebe benötigt, welche die Pumpen zum Fördern der Fluide oder die als Zentrifugen ausgestalteten Separatoren oder Rotationsfiltervorrichtungen oder Mischvorrichtungen antreiben.

In der Pharmaindustrie müssen bei der Herstellung von pharmazeutisch wirksamen Substanzen höchste Ansprüche an die Reinheit gestellt werden, oft müssen die mit den Substanzen in Kontakt kommenden Komponenten sogar steril sein. Ähnliche Anforderungen ergeben sich auch in der Biotechnologie, beispielsweise bei der Herstellung, Behandlung oder Züchtung von biologischen Substanzen, Zellen oder Mikroorganismen, wo ein extrem hohes Mass an Reinheit gewährleistet sein muss, um die Brauchbarkeit des hergestellten Produkts nicht zu gefährden.

Daher ist die Sterilität in derartigen Prozessen, in denen beispielsweise biologische Aktivitäten stattfinden, von sehr grosser Bedeutung. Das Sterilisieren der Vorrichtungen, beispielsweise mittels Dampfsterilisation, stellt sehr häufig einen zeit- und kostenintensiven Faktor dar. Deshalb besteht heute die zunehmende Tendenz, für solche biotechnologischen Prozesse Komponenten der jeweiligen Vorrichtung als Einmalteile auszugestalten, um aufwändige Sterilisationsprozesse zu vermeiden oder auf ein Minimum zu reduzieren. Insbesondere werden diejenigen Komponenten, die während des Prozesses mit den biologischen Substanzen in direkten Kontakt kommen, häufig als Einmalteile ausgestaltet. Der Begriff Einmalteile (single use) bezeichnet dabei Teile bzw. Komponenten, die bestimmungsgemäss nur einmal benutzt werden dürfen. Nach der Anwendung werden die Einmalteile entsorgt und für die nächste Anwendung durch neue, das heisst noch nicht gebrauchte Einmalteile ersetzt.

Diese Einmalteile werden vor ihrer Verwendung sterilisiert, beispielsweise durch Beaufschlagung mit Gamma-Strahlung. Diese Einmalteile oder Einmalvorrichtungen müssen dann mit anderen Komponenten beispielsweise einer für den Mehrfachgebrauch ausgestalteten wiederverwendbaren Vorrichtung zusammengesetzt werden. Dabei ist es natürlich äusserst wichtig, dass die Sterilität der Einmalteile bei dem Zusammensetzen mit der wiederverwendbaren Vorrichtung erhalten bleibt. Daher ist man bestrebt, die Einmalvorrichtungen und die wiederverwendbaren Vorrichtungen so auszugestalten, dass sie in möglichst einfacher Weise zusammengefügt bzw. voneinander getrennt werden können.

Aus der EP 3 115 103 A1 ist beispielsweise eine Mischvorrichtung mit einem Flügelrad bekannt, die eine Einmalvorrichtung und eine wiederverwendbare Vorrichtung umfasst, wobei der Rotor des elektromagnetischen Drehantriebs, welcher das Flügelrad antreibt, als Einmalteil ausgestaltet ist, und der Stator, mit welchem die Rotation des Rotors angetrieben wird, als wiederverwendbare Vorrichtung ausgestaltet ist.

Aus der DE 10 2020 121 422 ist ein Separator bekannt, mit welchem eine Suspension durch Zentrifugieren in zwei Phasen unterschiedlicher Dichte aufgeteilt wird. Der Separatoreinsatz mit dem Rotor zum Zentrifugieren der Suspension ist als Einmalteil ausgestaltet und kann in eine wiederverwendbare Vorrichtung eingesetzt werden, welche zwei axial beabstandet angeordnete Statoren umfasst, mit welcher der Rotor berührungslos magnetisch lagerbar und berührungslos zur Rotation antreibbar ist. Dabei ist im zusammengesetzten Zustand an den beiden axialen Enden des Rotors jeweils ein Stator vorgesehen, welcher einen jeweils im Rotor angeordneten Permanentmagneten umschliesst. Mindestens einer der Statoren wirkt dann mit dem von ihm umschlossenen Permanentmagneten als elektromagnetischer Drehantrieb zusammen, welcher die Rotation des Rotors antreibt. Der andere Stator wird nur für die berührungslose Lagerung des Rotors oder sowohl für die Lagerung als auch für den Antrieb des Rotors verwendet.

Zum Auswechseln des Separatoreinsatzes werden die beiden Statoren in axialer Richtung voneinander wegbewegt, sodass der Separatoreinsatz entnommen und durch einen neuen ersetzt werden kann. Dann müssen die Schläuche, durch welche die Fluide in den Separatoreinsatz eingebracht bzw. aus dem Separatoreinsatz abgeführt werden, durch eine zentrale Durchführung im jeweiligen Stator hindurch gefädelt werden. Anschliessend werden die beiden Statoren wieder aufeinander zubewegt, sodass der Separatoreinsatz bezüglich des Gehäuses der Vorrichtung zwischen den beiden Statoren fixiert ist.

Nachteilig an einer solchen Ausgestaltung ist es, dass die Einmalvorrichtung erst zusammengesetzt werden kann, wenn der Schlauch oder die Schläuche durch die Durchführungen in den Statoren eingefädelt ist/sind, weil die Schläuche üblicherweise mit anderen Einmalkomponenten verbunden sind, die üblicherweise nicht durch die Durchführung im Stator hindurch passen. Dieses Zusammensetzen der Einmalkomponenten der Einmalvorrichtung ist ein zusätzlicher Arbeitsschritt, der auch die Sterilität des gesamten Systems gefährdet oder zusätzliche, meist aufwändige Massnahmen bedingt, um die Sterilität oder die Reinheitsanforderungen des Prozesses zu gewährleisten.

Ausgehend von diesem Stand der Technik ist es daher eine Aufgabe der Erfindung, einen elektromagnetischen Drehantrieb vorzuschlagen, welcher eine Einmalvorrichtung für den Einmalgebrauch und eine wiederverwendbare Vorrichtung für den Mehrfachgebrauch umfasst, wobei die Einmalvorrichtung in besonders einfacher Weise mit der wiederverwendbaren Vorrichtung zusammensetzbar ist. Ferner ist es eine Aufgabe der Erfindung, eine Einmalvorrichtung für einen solchen Drehantrieb vorzuschlagen.

Die diese Aufgabe lösenden Gegenstände der Erfindung sind durch die Merkmale des unabhängigen Patentanspruchs der jeweiligen Kategorie gekennzeichnet.

Erfindungsgemäss wird also ein elektromagnetischer Drehantrieb vorgeschlagen mit einer Einmalvorrichtung, die für den Einmalgebrauch ausgestaltet ist, und mit einer wiederverwendbaren Vorrichtung, die für den Mehrfachgebrauch ausgestaltet ist, wobei die Einmalvorrichtung ein Rotorgehäuse mit einem Rotor, einen Verbindungsschlauch für ein Fluid, und mindestens eine weitere Einmalkomponente umfasst, wobei das Rotorgehäuse einen Einlass und einen Auslass aufweist, wobei der Rotor in dem Rotorgehäuse zum Rotieren um eine Solldrehachse vorgesehen ist, welche eine axiale Richtung definiert, wobei der Rotor berührungslos magnetisch antreibbar ist, und wobei der Verbindungsschlauch den Einlass oder den Auslass des Rotorgehäuses mit der weiteren Einmalkomponente verbindet, derart, dass im Betriebszustand das Fluid den Verbindungsschlauch durchströmen kann, wobei die wiederverwendbare Vorrichtung einen Stator umfasst, mit welchem der Rotor im Betriebszustand berührungslos magnetisch zur Rotation um die Solldrehachse antreibbar ist, wobei der Stator eine Mehrzahl von Wicklungen zur Erzeugung eines elektromagnetischen Drehfelds zum Antreiben des Rotors umfasst, und wobei der Stator ein Statorgehäuse mit einer Durchführung für den Verbindungsschlauch aufweist. Der Stator ist öffenbar mit einem ersten Statorteil und einem zweiten Statorteil ausgestaltet, welche relativ zueinander bewegbar sind, wobei in einer Offenstellung des Stators der Verbindungsschlauch in die Durchführung einlegbar ist, und wobei in einer Schliessstellung des Stators die Durchführung den Verbindungsschlauch umschliesst.

Diese Ausgestaltung mit dem öffenbaren Stator hat den Vorteil, dass die gesamte bereits zusammengesetzte Einmalvorrichtung mit der wiederverwendbaren Vorrichtung zusammengesetzt werden kann. Der Verbindungsschlauch kann also schon vor dem Zusammensetzen der Einmalvorrichtung und der wiederverwendbaren Vorrichtung einerseits mit der Einmalkomponente und andererseits mit dem Rotorgehäuse verbunden sein. Der bei bekannten Vorrichtungen zusätzliche Schritt, den Verbindungsschlauch erst durch die Durchführung im Statorgehäuse zu fädeln und ihn dann erst mit der Einmalkomponente und/oder mit dem Rotorgehäuse zu verbinden, entfällt bei der erfindungsgemässen Ausgestaltung.

Zum Zusammenfügen der Einmalvorrichtung mit der wiederverwendbaren Vorrichtung wird der Stator in die Offenstellung gebracht, der Verbindungsschlauch in die nun lateral geöffnete Durchführung eingelegt, und anschliessend der Stator in die Schliessstellung gebracht. Nun wird der Stator in die Schliessstellung gebracht, und die Durchführung umschliesst den Verbindungsschlauch, das heisst, der Verbindungsschlauch ist entlang seines gesamten Umfangs von der Durchführung umgeben.

Sinngemäss Gleiches gilt natürlich auch für das Trennen der Einmalvorrichtung von der wiederverwendbaren Vorrichtung. Wenn der Stator in der Offenstellung ist, kann die Einmalvorrichtung gesamthaft von der wiederverwendbaren Vorrichtung getrennt werden, ohne dass die Einmalvorrichtung vorgängig zerlegt oder Verbindungen zwischen Komponenten der Einmalvorrichtung gelöst werden müssen. Dies ist insbesondere auch unter hygienischen Aspekten ein erheblicher Vorteil.

Die Möglichkeit, den Verbindungsschlauch in der Offenstellung des Stators in die Durchführung einzulegen bzw. den Verbindungsschlauch aus der Durchführung zu entnehmen, ist auch im Hinblick auf die Sterilität ein grosser Vorteil. Da die Einmalvorrichtung gesamthaft in die wiederverwendbare Vorrichtung einsetzbar ist, entfällt das Erfordernis, verschiedene Bestandteile der Einmalvorrichtung erst dann miteinander zu verbinden, wenn die Einmalvorrichtung in die wiederverwendbare Vorrichtung eingesetzt ist. Hierdurch werden Berührungen oder Kontakte mit den Bestandteilen der Einmalvorrichtung auf ein Minimum reduziert. Montageschritte, um Bestandteile der Einmalvorrichtung nach dem Einsetzen in die wiederverwendbare Vorrichtung miteinander zu verbinden, entfallen, wodurch sich das Erhalten der Sterilität deutlich vereinfacht.

Durch die erfindungsgemässe Ausgestaltung ist es somit möglich, die Einmalvorrichtung als vormontierte Einheit im zusammengesetzten Zustand gesamthaft in die wiederverwendbare Vorrichtung einzusetzen bzw. von der wiederverwendbaren Vorrichtung zu trennen. Die Einmalvorrichtung kann also als vollständig assemblierte Einheit ausgestaltet werden. Ferner kann die Einmalvorrichtung als vollständig assemblierte Einheit sterilisiert werden. Somit kann die vollständig assemblierte Einmalvorrichtung sterilisiert in einer Verpackung eingeschlossen sein. Natürlich kann die in der Verpackung eingeschlossene, vollständig assemblierte Einmalvorrichtung auch in der Verpackung sterilisiert werden, beispielsweise durch Beaufschlagung mit Gammastrahlung.

Gemäss einer bevorzugten Ausgestaltung sind der erste Statorteil und der zweite Statorteil mittels eines Gelenks miteinander verbunden. Die gelenkige Verbindung der beiden Statorteile stellt eine besonders einfache Massnahme da, um den Stator aus der Offenstellung in die Schliessstellung zu bringen bzw. umgekehrt aus der Schliessstellung in die Offenstellung. Der Stator kann also in einfacher Weise aufgeklappt und zugeklappt werden. Wenn der Stator aufgeklappt ist, kann der Verbindungsschlauch in die Durchführung eingelegt werden. Wenn der Stator dann zugeklappt wird, umschliesst die Durchführung den Verbindungsschlauch.

Vorzugsweise ist eine elektrische Verbindung vorgesehen, welche durch das Gelenk hindurchgeführt ist. Dadurch kann der Stator auf- und zugeklappt werden, ohne dass die Gefahr der Beschädigung von elektrischen Verbindungen besteht.

Ferner ist es bevorzugt, dass am Statorgehäuse eine mechanische Fixierung vorgesehen ist, mit welcher in der Schliessstellung der erste Statorteil lösbar am zweiten Statorteil fixiert ist. Die mechanische Fixierung kann beispielsweise einen Spannverschluss oder eine Mehrzahl von Spannverschlüssen umfassen. Jeder Spannverschluss ist dann in an sich bekannter Weise so am Statorgehäuse angeordnet, dass er die Stossstelle zwischen den beiden Statorteilen übergreift. Die Stossstelle ist diejenige Stelle, an welcher die beiden Statorteile in der Schliessstellung aneinandergrenzen und in der Offenstellung voneinander entfernt sind.

Eine weitere vorteilhafte Massnahme besteht darin, dass eine mechanische Zentrierung vorgesehen ist, welches in der Schliessstellung des Stators den ersten Statorteil relativ zu dem zweiten Statorteil zentriert. Diese mechanische Zentrierung kann beispielsweise eine an einem der beiden Statorteile vorgesehene Ausnehmung umfassen, sowie eine an dem anderen der beiden Statorteile vorgesehenen Erhebung, wobei die Ausnehmung und die Erhebung so ausgestaltet sind, dass die Erhebung in der Schliessstellung des Stators in die Ausnehmung eingreift.

Gemäss einer bevorzugten Ausgestaltung ist das Statorgehäuse im Wesentlichen zylinderförmig ausgestaltet, wobei der erste Statorteil in Umfangsrichtung gesehen länger ausgestaltet ist als der zweite Statorteil. Wenn der Stator beispielsweise mit einer ungraden Anzahl von Statorpolen ausgestaltet ist, dann umfasst einer der beiden Statorteile mehr Statorpole als der andere Statorteil.

Natürlich sind auch solche Ausgestaltungen möglich, bei denen die beiden Statorteile im Wesentlichen gleich ausgestaltet sind, also beispielsweise beide Statorteile gleich viele Statorpole umfassen.

Ferner sind solche Ausgestaltungen bevorzugt, bei denen in jedem Statorteil mindestens eine Wicklung vorgesehen ist, wobei die Anzahl der Wicklungen in dem ersten Statorteil verschieden ist von der Anzahl der Wicklungen in dem zweiten Statorteil.

Natürlich sind auch solche Ausgestaltungen möglich, bei denen die beiden Statorteile gleich viele Wicklungen umfassen

Vorzugsweise ist der Stator als Lager- und Antriebsstator ausgestaltet, mit welchem der Rotor im Betriebszustand berührungslos magnetisch bezüglich des Stators lagerbar ist. Diese Ausgestaltung ermöglicht eine besonders kostengünstige und auch platzsparende, kompakte Ausgestaltung, weil der Stator nicht nur als Antriebsstator ausgestaltet ist, sondern gleichzeitig der Stator für die magnetische Lagerung des Rotors ist.

Gemäss einer bevorzugten Ausgestaltung umfasst der Rotor einen magnetisch wirksamen Kern, und der Stator weist eine Mehrzahl von Statorpolen auf, die um den magnetisch wirksamen Kern herum angeordnet sind, und von denen jeder jeweils durch eine dem magnetisch wirksamen Kern des Rotors zugewandte Stirnfläche begrenzt wird.

Vorzugsweise ist der elektromagnetische Drehantrieb als Tempelmotor ausgestaltet, wobei der Stator eine Mehrzahl von Spulenkernen aufweist, von denen jeder einen stabförmigen Längsschenkel umfasst, welcher sich von einem ersten Ende in axialer Richtung bis zu einem zweiten Ende erstreckt, sowie einen Querschenkel, welcher an dem zweiten Ende des Längsschenkels angeordnet ist, und welcher sich in einer radialen Richtung erstreckt, die senkrecht zu der axialen Richtung ist, wobei jeder Querschenkel einen der Statorpole bildet, und wobei an jedem Längsschenkel mindestens eine konzentrierte Wicklung angeordnet ist, welche den jeweiligen Längsschenkel umgibt.

In einem bevorzugten Ausführungsbeispiel erstreckt sich der Rotor in axialer Richtung von einem ersten Ende bis zu einem zweiten Ende, wobei an jedem Ende ein magnetisch wirksamer Kern vorgesehen ist. Ferner umfasst der Drehantrieb bei diesem Ausführungsbeispiel zwei Statoren, die bezüglich der axialen Richtung beabstandet angeordnet sind, und von denen jeder erfindungsgemäss ausgestaltet ist, wobei jeder Stator zum Zusammenwirken mit genau einem der magnetisch wirksamen Kerne angeordnet und ausgestaltet ist, derart dass der Rotor im Betriebszustand berührungslos magnetisch bezüglich der Statoren lagerbar ist.

Eine solche Ausgestaltung des elektromagnetischen Drehantriebs eignet sich beispielsweise für Separatoren oder andere Vorrichtungen, bei denen der Rotor eine grosse Erstreckung in axialer Richtung aufweist, sodass er mit nur einer magnetischen Lagerstelle nicht mehr zuverlässig berührungslos magnetisch lagerbar ist. Es werden dann zwei magnetische Lagerstellen für den Rotor vorgesehen, nämlich eine am ersten Ende des Rotors und eine am zweiten Ende des Rotors. Die dort vorgesehenen magnetisch wirksamen Kerne wirken jeweils mit einem der beiden Statoren zusammen, sodass der Rotor im Betriebszustand mittels dieser beiden Lagerstellen berührungslos magnetisch bezüglich des Stators lagerbar ist. Die beiden Statoren können, müssen aber nicht identisch ausgestaltet sein.

Bei dieser Ausgestaltung mit zwei Statoren ist es auch möglich, nur einen der beiden Statoren für den Antrieb des Rotors auszugestalten oder zu verwenden. Es ist aber auch möglich, jeden der beiden Statoren sowohl für den Antrieb als auch für die berührungslose magnetische Lagerung des Rotors auszugestalten oder zu verwenden.

Vorzugsweise ist eine Kontrolleinrichtung vorgesehen, die an oder in dem ersten oder dem zweiten Statorteil angeordnet ist. Die Kontrolleinrichtung umfasst typischerweise die Leistungselektronik zur Versorgung und zur Ansteuerung der Wicklungen, mit denen die elektromagnetischen Felder erzeugt werden, und Komponenten für die Signalverarbeitung, beispielsweise die Verarbeitung von Daten die von Sensoren, wie Positionssensoren für die Erfassung der Rotorposition, erfasst werden. Dabei ist es bei jedem Stator möglich, dass die Kontrolleinrichtung nur auf oder an dem ersten Statorteil angeordnet ist, oder nur auf oder an dem zweiten Statorteil, oder dass die Kontrolleinrichtung auf den ersten und den zweiten Statorteil verteilt ist.

Durch die Erfindung wird ferner eine Einmalvorrichtung für einen elektromagnetischen Drehantrieb vorgeschlagen, der erfindungsgemäss ausgestaltet ist. Die Einmalvorrichtung umfasst das Rotorgehäuse mit dem Rotor, die Einmalkomponente und den Verbindungsschlauch.

Da bei der erfindungsgemässen Ausgestaltung die Einmalvorrichtung gesamthaft, also im zusammengesetzten Zustand, in die wiederverwendbare Vorrichtung einsetzbar ist, sind vorzugsweise alle Bestandteile der Einmalvorrichtung zu einer vormontierten Einheit zusammengesetzt, welche gesamthaft in die wiederverwendbare Vorrichtung einsetzbar ist.

Vorzugsweise ist die Einmalvorrichtung sterilisiert in einer Verpackung eingeschlossen. Dabei ist es natürlich auch möglich, dass die Einmalvorrichtung zunächst in der Verpackung eingeschlossen wird, und erst zu einem späteren Zeitpunkt, beispielsweise kurz vor der Auslieferung an den Verwender oder vom Verwender selbst, durch die Verpackung hindurch sterilisiert wird.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der Zeichnung zeigen (teilweise im Schnitt):
- Fig. 1:: eine schematische Darstellung eines ersten Ausführungsbeispiels eines erfindungsgemässen elektromagnetischen Drehantriebs,
- Fig. 2:: eine perspektivische Darstellung des Stators und des Rotorgehäuses des ersten Ausführungsbeispiels in der Offenstellung des Stators (ohne Statorgehäuse),
- Fig. 3:: eine perspektivische Darstellung eines zweiten Ausführungsbeispiels eines erfindungsgemässen elektromagnetischen Drehantriebs,
- Fig. 4:: eine schematische Darstellung des Stators des zweiten Ausführungsbeispiels in der Offenstellung (links) und in der Schliessstellung(rechts), jeweils in einem Querschnitt senkrecht zur axialen Richtung entlang der Schnittlinie IV-IV in Fig. 3,
- Fig. 5:: eine perspektivische Darstellung einer Ausführungsform des Statorgehäuses in der Schliessstellung,
- Fig. 6:: das Statorgehäuse aus Fig. 5 in der Offenstellung,
- Fig. 7: eine Schnittdarstellung des Gelenks des Statorgehäuses aus Fig. 5 in einem Schnitt in axialer Richtung,
- Fig. 8:: eine perspektivische Darstellung eines dritten Ausführungsbeispiels eines erfindungsgemässen elektromagnetischen Drehantriebs, wobei der Stator in der Offenstellung ist,
- Fig. 9:: wie Fig. 8, jedoch mit dem Stator in der Schliessstellung,
- Fig. 10:: eine perspektivische Darstellung eines vierten Ausführungsbeispiels eines erfindungsgemässen elektromagnetischen Drehantriebs, wobei der Stator in der Offenstellung ist,
- Fig. 11:: eine perspektivische Darstellung einer ersten Variante für die Ausgestaltung des Stators, wobei der Stator in der Schliessstellung ist,
- Fig. 12:: den Stator aus Fig. 11 in der Offenstellung,
- Fig. 13:: eine perspektivische Darstellung einer Variante für die Ausführung des Statorgehäuses (in der Offenstellung)
- Fig. 14:: eine perspektivische Darstellung einer zweiten Variante für die Ausgestaltung des Stators, wobei der Stator in der Schliessstellung ist,
- Fig. 15:: eine perspektivische Darstellung einer dritten Variante für die Ausgestaltung des Stators und des Rotors, wobei der Stator in der Schliessstellung ist,
- Fig. 16:: wie Fig. 15, jedoch mit dem Stator in der Offenstellung,
- Fig. 17:: eine perspektivische Darstellung einer vierten Variante für die Ausgestaltung des Stators, wobei der Stator in der Schliessstellung ist,
- Fig. 18:: wie Fig. 17, jedoch mit dem Stator in der Offenstellung,
- Fig. 19:: eine perspektivische Darstellung einer fünften Variante für die Ausgestaltung des Stators und des Rotors, wobei der Stator in der Schliessstellung ist,
- Fig. 20:: wie Fig. 19, jedoch mit dem Stator in der Offenstellung,
- Fig. 21:: eine perspektivische Darstellung einer sechsten Variante für die Ausgestaltung des Stators und des Rotors, wobei der Stator in der Schliessstellung ist,
- Fig. 22:: wie Fig. 21, jedoch mit dem Stator in der Offenstellung, und
- Fig. 23-25:: verschiedene Varianten für die Ausgestaltung der Kontrolleinrichtung, jeweils in einer schematischen Darstellung.

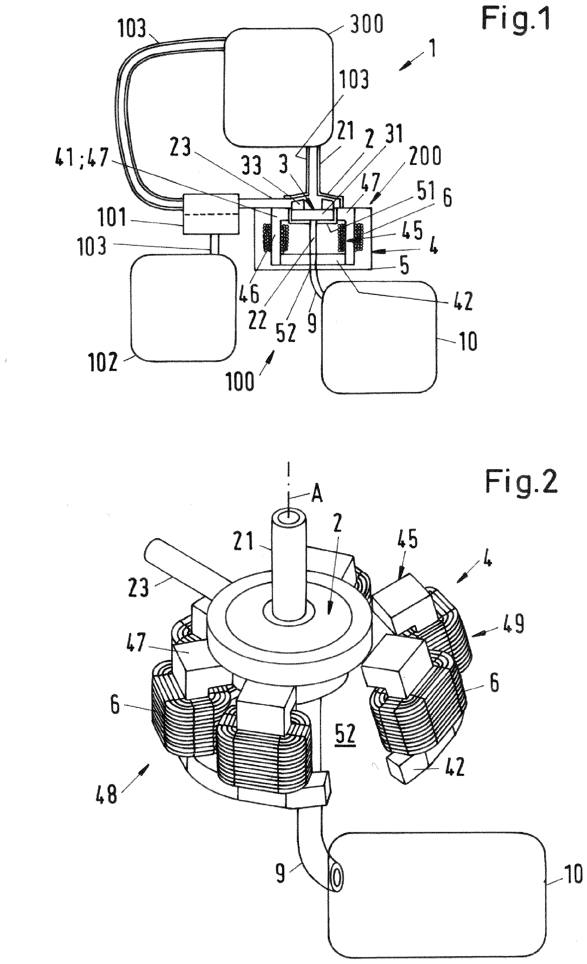

Fig. 1 zeigt eine schematische Darstellung eines ersten Ausführungsbeispiels eines erfindungsgemässen elektromagnetischen Drehantriebs, der gesamthaft mit dem Bezugszeichen 1 bezeichnet ist. In der folgenden Beschreibung wird mit beispielhaftem Charakter auf den Anwendungsfall Bezug genommen, dass der erfindungsgemässe Drehantrieb 1 für biotechnologische oder pharmazeutische Prozesse verwendet wird, bei denen ein sehr hohes Mass an Reinheit oder Sterilität derjenigen Komponenten wesentlich ist, die mit den im Prozess befindlichen Substanzen in Kontakt kommen.

Der elektromagnetische Drehantrieb 1 kann für eine Vielzahl von Vorrichtungen ausgestaltet sein, die eine rotierende Komponente benötigen, beispielsweise für Zentrifugalpumpen, mit denen fliessfähige Substanzen gefördert oder zirkuliert werden, oder für Mischer, mit denen z.B. eine kontinuierliche Durchmischung und eine Zirkulation der Nährflüssigkeit in einem Bioreaktor realisiert wird, oder für Separatoren, die nach dem Prinzip einer Zentrifuge arbeiten, und mit denen fliessfähige Substanzen wie Suspensionen in zwei Phasen unterschiedlicher Dichte aufgeteilt werden, oder für Filtervorrichtungen, insbesondere Rotationsfilter, mit denen z.B. Stoffwechselprodukte von Zellen von einem Fluid separiert werden.

Ein wesentlicher Aspekt des erfindungsgemässen Drehantriebs 1 ist es, dass der elektromagnetische Drehantrieb 1 eine Einmalvorrichtung 100 umfasst, die für den Einmalgebrauch ausgestaltet ist, und eine wiederverwendbare Vorrichtung 200, die für den Mehrfachgebrauch, also für den dauerhaften Gebrauch, ausgestaltet ist. Dabei umfasst die Einmalvorrichtung 100 vorzugsweise diejenigen Komponenten, die mit den Substanzen im jeweiligen Prozess in Kontakt kommen.

Mit dem Begriff "Einmalvorrichtung" und anderen Zusammensetzungen mit dem Bestandteil "Einmal", wie z. B. Einmalteil, Einmalkomponente usw., sind dabei solche Vorrichtungen, Komponenten bzw. Teile gemeint, die für den Einmalgebrauch ausgestaltet sind, die also bestimmungsgemäss nur ein einziges Mal benutzt werden können und dann entsorgt werden. Für eine neue Anwendung muss dann ein neues, bisher unbenutztes Einmalteil eingesetzt werden. Bei der Konzipierung bzw. der Ausgestaltung der Einmalvorrichtung 100 sind es daher wesentliche Aspekte, dass die Einmalvorrichtung 100 möglichst einfach und wirtschaftlich herstellbar ist, wenige Kosten verursacht und aus möglichst preisgünstig erhältlichen Materialen herstellbar ist. Ein anderer wesentlicher Aspekt ist es, dass die Einmalvorrichtung 100 in möglichst einfacher Weise mit der wiederverwendbaren Vorrichtung 200 zu dem elektromagnetischen Drehantrieb 1 zusammenfügbar ist. Die Einmalvorrichtung 100 soll also in sehr einfacher Weise ersetzt werden können, ohne dass dafür ein hoher Montageaufwand notwendig ist. Besonders bevorzugt soll die Einmalvorrichtung 100 ohne die Verwendung von Werkzeugen mit der wiederverwendbaren Vorrichtung 200 zusammenfügbar sein und von der wiederverwendbaren Vorrichtung 200 trennbar sein. Bei sterilen Anwendungen soll die Sterilität der Einmalvorrichtung 100 durch das Zusammenfügen mit der wiederverwendbaren Vorrichtung nicht verloren gehen.

Bei dem elektromagnetischen Drehantrieb 1 (Fig. 1) umfasst die Einmalvorrichtung 100 mindestens die folgenden Komponenten: ein Rotorgehäuse 2 mit einem darin angeordneten Rotor 3, einen Verbindungsschlauch 9 für ein Fluid, und mindestens eine weitere Einmalkomponente 10, beispielsweise ein Reservoir oder ein Vorratsbehälter für ein Fluid. Das Rotorgehäuse 2 umfasst mindestens einen Einlass 21 und einen Auslass 22, durch welche das Fluid in das Rotorgehäuse 2 einbringbar bzw. aus dem Rotorgehäuse 2 abführbar ist.

Der Rotor 3 in dem Rotorgehäuse 2 ist zum Rotieren um eine Solldrehachse ausgestaltet, welche eine axiale Richtung A (Fig. 2) definiert, wobei der Rotor 3 berührungslos magnetisch antreibbar ist. Dazu umfasst der Rotor 3 einen magnetisch wirksamen Kern 31. Mit dem "magnetisch wirksamen Kern 31" des Rotors 3 ist derjenige Bereich des Rotors 3 gemeint, welcher für die Drehmomentbildung sowie für die Erzeugung der magnetischen Lagerkräfte magnetisch mit einem Stator 4 zusammenwirkt.

Der Verbindungsschlauch 9 verbindet den Einlass 21 oder den Auslass 22 des Rotorgehäuses 2 mit der Einmalkomponente 10, derart, dass im Betriebszustand das Fluid den Verbindungsschlauch 9 durchströmen kann. Bei dem in Fig. 1 dargestellten Ausführungsbeispiel verbindet der Verbindungsschlauch 9 den Auslass des Rotorgehäuses 2 mit der Einmalkomponente 10.

Die wiederverwendbare Vorrichtung umfasst den Stator 4, mit welchem der Rotor 3 im Betriebszustand berührungslos magnetisch zur Rotation um die Solldrehachse antreibbar ist, wobei der Stator 4 eine Mehrzahl von Wicklungen 6 zur Erzeugung eines elektromagnetischen Drehfelds zum Antreiben des Rotors 3 umfasst. Der Stator 4 ist in einem Statorgehäuse 5 angeordnet, welches als Umkapslung des Stators 4 ausgestaltet ist, vorzugsweise als hermetische Umkapslung.

Das Statorgehäuse 5 wird bezüglich der axialen Richtung A von zwei Stirnflächen begrenzt. In einer der beiden Stirnflächen ist eine zentrale Ausnehmung 51 vorgesehen, in welche das Rotorgehäuse 2 einsetzbar ist, sodass der im Rotorgehäuse 2 angeordnete Rotor 3 magnetisch mit dem Stator wechselwirken kann, und mittels der Wicklungen 6 des Stators 4 berührungslos zur Rotation antreibbar ist.

Das Statorgehäuse 5 umfasst ferner eine Durchführung 52 für den Verbindungsschlauch 9, welche sich von der zentralen Ausnehmung 51 durch das Statorgehäuse 5 bis zu der anderen Stirnfläche erstreckt, also der Stirnfläche, welche der zentralen Ausnehmung abgewandt ist. Vorzugsweise, aber nicht notwendigerweise, erstreckt sich die Durchführung 52 in axialer Richtung A. Ferner ist es bevorzugt, dass die Durchführung 52 zentral im Statorgehäuse 5 angeordnet ist.

Die Durchführung 52 dient dazu, dass der Verbindungsschlauch 9, von dem Einlass 21 oder dem Auslass 22 des Rotorgehäuses 2 - hier dem Auslass 22 - durch das Statorgehäuse 5 hindurch nach aussen zu der Einmalkomponente 10 geführt werden kann, um so eine Strömungsverbindung für das Fluid zwischen dem Rotorgehäuse 2 und der Einmalkomponente 10 zu bilden.

Zum besseren Verständnis zeigt Fig. 2 eine perspektivische Darstellung des Stators 4, des Rotorgehäuses 2, der Verbindungsleitung 9 und der Einmalkomponente 10 des ersten Ausführungsbeispiels. In Fig. 2 ist zur besseren Übersicht das Statorgehäuse 5 des Stators 4 nicht dargestellt.

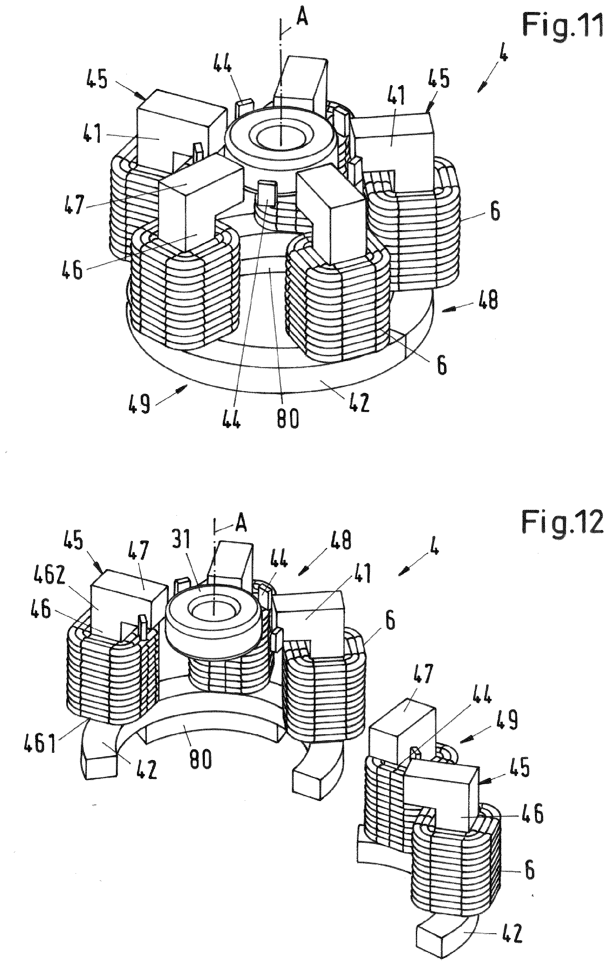

Bei dem ersten Ausführungsbeispiel ist der elektromagnetische Drehantrieb 1 als Tempelmotor ausgestaltet. Die Ausgestaltung als Tempelmotor wird weiter hinten anhand der Fig. 11 und 12 noch näher erläutert.

Bei einem Tempelmotor umfasst den Stator 4 eine Mehrzahl von Spulenkernen 45, von denen jeder einen stabförmigen Längsschenkel 46 umfasst, welcher sich von einem ersten Ende 461 (z. B. Fig. 11) in axialer Richtung A bis zu einem zweiten Ende 462 erstreckt, sowie einen Querschenkel 47, welcher an dem zweiten Ende 462 des Längsschenkels 46 angeordnet ist. Jeder Querschenkel 47 erstreckt sich in radialer Richtung auf den Rotor 3 zu. Die radiale Richtung ist eine zur axialen Richtung A senkrechte Richtung.

Die ersten Enden 461 aller Längsschenkel sind über einen Rückschluss 42 magnetisch leitend miteinander verbunden.

Jeder Spulenkern 45 hat also eine L-förmige Ausgestaltung, wobei die Längsschenkel 46 jeweils den sich in axialer Richtung A erstreckenden langen Schenkel des L bilden, und die sich senkrecht zu den Längsschenkeln 46 in radialer Richtung auf den Rotor 3 hin gerichtet erstreckenden Querschenkel 47 jeweils den kurzen Schenkel des L bilden. Die Mehrzahl der stabförmigen Längsschenkel 46, die sich in axialer Richtung A erstrecken und an die Säulen eines Tempels erinnern, hat dem Tempelmotor seinen Namen gegeben.

Jeder Querschenkel 47 bildet jeweils einen Statorpol 41. Die Statorpole 41 sind um den magnetisch wirksamen Kern 31 des Rotors 3 herum angeordnet, das heisst, im Betriebszustand ist der magnetisch wirksame Kern 31 des Rotors 3 von den Statorpolen 41 umgeben. Die Wicklungen 6, die vorzugsweise als konzentrierte Wicklungen 6 ausgestaltet sind, sind an den Längsschenkeln 46 angeordnet. An jedem Längsschenkel 46 ist mindestens eine der konzentrierten Wicklungen 6 angeordnet, welche den jeweiligen Längsschenkel 46 umgibt.

In diesem Sinne ist im Rahmen der vorliegenden Anmeldung der Begriff Tempelmotor zu verstehen.

Erfindungsgemäss ist der Stator 4 des elektromagnetischen Drehantriebs 1 öffenbar mit einem ersten Statorteil 48 und einem zweiten Statorteil 49 ausgestaltet, welche relativ zueinander bewegbar sind, wobei in einer Offenstellung des Stators 4 der Verbindungsschlauch 9 in die Durchführung 52 einlegbar ist, und wobei in einer Schliessstellung des Stators 4 die Durchführung 52 den Verbindungsschlauch 9 umschliesst.

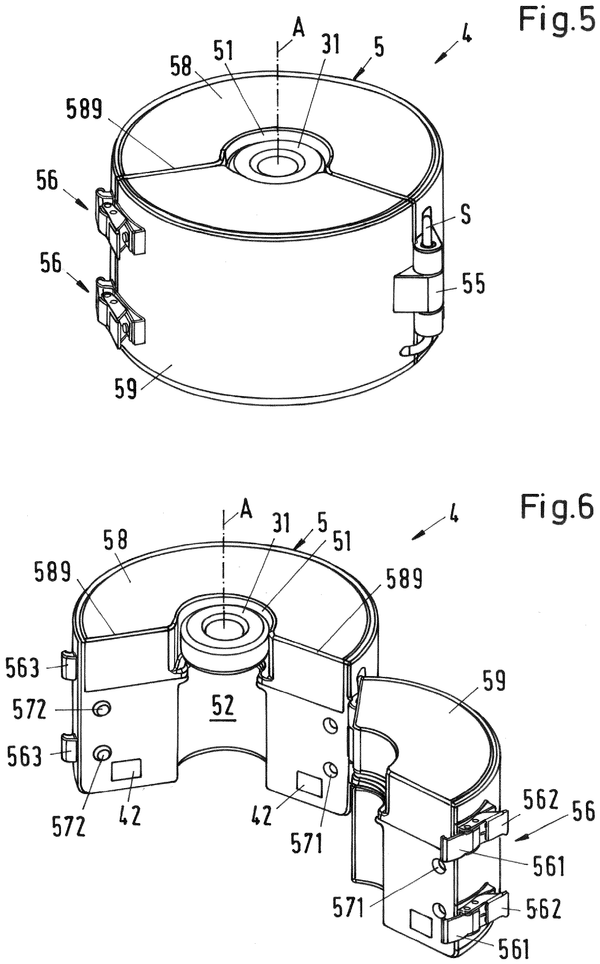

Vorzugsweise sind dabei der erste Statorteil 48 und der zweite Statorteil 49 mittels eines Gelenks 55 (z.B. Fig. 5) miteinander verbunden. Das Gelenk 55 ist bevorzugt am Statorgehäuse 5 angeordnet, wie weiter hinten noch genauer erläutert wird, beispielsweise anhand der Fig. 5 und Fig. 6. Der Stator 4 ist dann als Klappstator 4 ausgestaltet, der aus der Schliessstellung in die Offenstellung aufgeklappt und aus der Offenstellung in die Schliessstellung zugeklappt werden kann.

Fig. 2 zeigt den Stator des ersten Ausführungsbeispiels in der Offenstellung. Wie das gut zu erkennen ist, kann in der Offenstellung des Stators 4 der Verbindungsschlauch 9 von der Seite, also beispielsweise in radialer Richtung, in die Durchführung 52 eingelegt werden. Anschliessend wird der Stator 4 in die Schliessstellung gebracht, in welcher die Durchführung 52 den Verbindungsschlauch 9 entlang seines gesamten Umfangs umgibt.

Im Unterschied zum Stand der Technik ist es also nicht mehr notwendig, den Verbindungsschlauch 9 durch die Durchführung 52 zu fädeln, sondern der Verbindungsschlauch 9 kann in einfacher Weise in den Stator 4 in der Offenstellung eingelegt werden. Wenn der Stator 4 dann in die Schliessstellung gebracht wird, ist der Verbindungsschlauch von der Durchführung 52 umschlossen.

Dies hat insbesondere den Vorteil, dass die Einmalvorrichtung 100 bereits vollständig assembliert sein kann, bevor sie in die wiederverwendbare Vorrichtung eingesetzt wird. Somit kann die Einmalvorrichtung 100 zu einer vormontierten Einheit zusammengesetzt werden, die dann gesamthaft sterilisiert werden kann, und die gesamthaft im zusammengesetzten Zustand in einer Verpackung eingeschlossen sein kann. Für den Anwendungsfall wird dann die vollständig assemblierte Einmalvorrichtung 100 mit der wiederverwendbaren Vorrichtung 200 zusammengefügt, wobei der Verbindungsschlauch 9 in die Durchführung 52 eingelegt wird. Anschliessend wird der Stator 4 in sie Schliessstellung gebracht.

Dabei spielt es auch keine Rolle, dass die Einmalkomponente 10 üblicherweise solche Abmessungen hat, dass sie nicht durch die Durchführung 52 hindurchgeführt werden könnte, denn durch die Möglichkeit, den Verbindungsschlauch 9 in die Durchführung 52 einzulegen, wenn der Stator 4 in der Offenstellung ist, kann der Verbindungsschlauch 9 bereits vor dem Einlegen in die Durchführung 52 mit der Einmalkomponente 10 verbunden sein.

Im Folgenden werden in nicht abschliessender Aufzählung einige Beispiels genannt, als was die Einmalkomponente 10 ausgestaltet sein kann: Tank, Reservoir, Vorratsbehälter, Bioreaktor, Pumpeneinheit einer Pumpe, beispielsweise einer Zentrifugalpumpe, eine Mehrzahl von Schläuchen, Filtervorrichtung, beispielsweise ein Rotationsfilter oder die Filtereinrichtung eines Tangentialflussfilters, Mischer, Separatoreinsatz eines Separators, beispielsweise eines Zentrifugen-Separators, Sensoren, Verbindungselement für die Verbindung mit anderen Einmalkomponenten. Es versteht sich, dass die Einmalkomponente 10 auch nur als Teil einer der vorangehend genannten Beispiele ausgestaltet sein kann. Beispielsweise kann die Einmalkomponente 10 als ein faltbarerer Plastiksack ausgestaltet sein, der selbst nicht formstabil ist und dann in einen formstabilen Stützbehälter eingesetzt wird, um beispielsweise einen Bioreaktor zu realisieren.

Bei dem ersten Ausführungsbeispiel (Fig. 1) ist der elektromagnetische Drehantrieb 1 als Zentrifugalpumpe zum Fördern des Fluids ausgestaltet. Dazu ist der Rotor 3 des elektromagnetischen Drehantriebs 1 gleichzeitig als Rotor 3 der Zentrifugalpumpe zum Fördern des Fluids ausgestaltet.

Das Rotorgehäuse 2 ist als Pumpengehäuse ausgestaltet, welches den Einlass 21, den Auslass 22 und einen zweiten Auslass 23 aufweist. Der Einlass 21 ist als axialer Einlass 21 ausgestaltet, sodass das Fluid in axialer Richtung A in das Rotorgehäuse 2 einströmt. Der zweite Auslass 23 ist als radialer oder tangentialer Auslass 23 ausgestaltet und bildet den Hauptauslass, durch welchen der Hauptstrom des Fluids in radialer bzw. tangentialer Richtung aus dem Rotorgehäuse 2 abgeführt wird. Der Auslass 22 ist als axialer Auslass ausgestaltet und bezüglich der axialen Richtung A auf der anderen Seite des Rotors 3 wie der Einlass 21 im Rotorgehäuse 2 angeordnet. Durch den Auslass 22 kann ein Teil des geförderten Fluids durch den Verbindungsschlauch 9 in die Einmalkomponente 10, beispielsweise ein Vorratsbehälter, abgeführt werden.

Das als Pumpengehäuse ausgestaltete Rotorgehäuse 2 ist derart ausgestaltet, dass es so in die zentrale Ausnehmung 51 des Statorgehäuses 5 einsetzbar ist, dass der magnetisch wirksame Kern des Rotors 31 von den Statorpolen 41 umgeben wird.

Der Rotor 3 umfasst eine Mehrzahl von Flügeln 33 zum Fördern des Fluids, beispielsweise vier Flügel 33. Der Rotor 3 umfasst ferner eine nicht im Detail dargestellte Ummantelung, mit welcher der magnetisch wirksame Kern 31 des Rotors 3 umschlossen und vorzugsweise hermetisch eingekapselt ist, sodass der magnetisch wirksame Kern 31 des Rotors 3 nicht in Kontakt mit dem zu fördernden Fluid kommt. Alle Flügel 33 sind auf der Ummantelung angeordnet und bezüglich der Umfangsrichtung des Rotors 3 äquidistant angeordnet. Jeder Flügel 33 erstreckt sich in radialer Richtung nach aussen und ist drehfest mit der Ummantelung verbunden. Der Rotor 3 mit den Flügeln 33 bildet das Flügelrad bzw. das Laufrad der Zentrifugalpumpe, mit welchem auf das Fluid oder die Fluide eingewirkt wird.

Ein vorteilhafter Aspekt ist es, dass der Rotor 3 als Integralrotor ausgestaltet ist, weil er sowohl der Rotor 3 des elektromagnetischen Drehantriebs 1 ist, als auch der Rotor 3 der Zentrifugalpumpe, mit welchem das Fluid gefördert wird.

Besonders bevorzugt ist der Stator 4 des elektromagnetischen Drehantriebs 1 als Lager- und Antriebsstator ausgestaltet, mit welchen der Rotor 3 im Betriebszustand berührungslos magnetisch bezüglich des Stators 4 lagerbar ist.

Zudem ist der Rotor 3 - wie bereits erwähnt - mittels des Stators 4 berührungslos magnetisch zur Rotation um die Solldrehachse antreibbar. Die Solldrehachse bezeichnet dabei diejenige Achse, um welche sich der Rotor 3 im Betriebszustand dreht, wenn sich der Rotor 3 bezüglich des Stators 4 in einer zentrierten und unverkippten Lage befindet. Diese Solldrehachse definiert die axiale Richtung A. Üblicherweise stimmt die die axiale Richtung A festlegende Solldrehachse mit der Mittelachse des Stators 4 überein.

Vorzugsweise ist der elektromagnetische Drehantrieb 1 nach dem Prinzip des lagerlosen Motors ausgestaltet und wird nach diesem Prinzip betrieben. Mit dem Begriff 'lagerloser Motor' ist dabei ein elektromagnetischer Drehantrieb 1 gemeint, bei welchem der Rotor 3 vollkommen magnetisch bezüglich des Stators 4 gelagert ist, wobei keine separaten magnetischen Lager vorgesehen sind. Der Stator 4 ist dazu als Lager- und Antriebsstator ausgestaltet, der sowohl Stator 4 des elektrischen Antriebs als auch Stator 4 der magnetischen Lagerung ist. Mit den elektrischen Wicklungen 6 des Stators 4 lässt sich ein magnetisches Drehfeld erzeugen, welches zum einen ein Drehmoment auf den Rotor 3 ausübt, das dessen Rotation um die Solldrehachse bewirkt, und welches zum anderen eine beliebig einstellbare Querkraft in einer zur axialen Richtung A senkrechten radialen Ebene auf den Rotor 3 ausübt, sodass dessen radiale Position aktiv steuerbar bzw. regelbar ist. Somit sind drei Freiheitsgrade des Rotors 3 aktiv regelbar, nämlich seine Rotation sowie seine radiale Position (zwei Freiheitsgrade). Bezüglich dreier weiterer Freiheitsgrade, nämlich seiner Position in axialer Richtung A und Verkippungen bezüglich der zur Solldrehachse senkrechten radialen Ebene (zwei Freiheitsgrade), ist der Rotor passiv magnetisch, das heisst nicht ansteuerbar, durch Reluktanzkräfte gelagert bzw. stabilisiert. Das Nichtvorhandensein eines separaten magnetischen Lagers bei vollständiger magnetischer Lagerung des Rotors 3 ist die Eigenschaft, welcher der lagerlose Motor seinen Namen verdankt. In dem Lager- und Antriebsstator lässt sich die Lagerfunktion nicht von der Antriebsfunktion separieren.

Beim lagerlosen Motor werden im Unterschied zu klassischen Magnetlagern die magnetische Lagerung und der Antrieb des Motors über elektromagnetische Drehfelder realisiert. Typischerweise wird in dem lagerlosen Motor die magnetische Antriebs- und Lagerfunktion durch die Überlagerung zweier magnetischer Drehfelder generiert, die üblicherweise als Antriebs- und Steuerfeld bezeichnet werden. Diese beiden mit den Wicklungen 6 des Stators 4 erzeugten Drehfelder haben in der Regel eine Polpaarzahl, die sich um eins unterscheidet. Hat also beispielsweise das Antriebsfeld die Polpaarzahl p, so hat das Steuerfeld die Polpaarzahl p+1 oder p-1. Dabei werden mit dem Antriebsfeld auf den magnetisch wirksamen Kern 31 in der radialen Ebene wirkende Tangentialkräfte erzeugt, die ein Drehmoment bewirken, was die Rotation um die axiale Richtung A bewirkt. Durch die Überlagerung des Antriebsfelds und des Steuerfelds lässt sich zusätzlich eine beliebig einstellbare Querkraft auf den magnetisch wirksame Kern 31 in der radialen Ebene erzeugen, mit welcher die Position des magnetisch wirksamen Kerns 31 in der radialen Ebene regelbar ist. Es ist also nicht möglich, den elektromagnetischen Fluss, der von den konzentrierten Wicklungen 6 generiert wird, aufzuteilen in einen (elektro-) magnetischen Fluss, der nur für den Antrieb der Rotation sorgt und einen (elektro-) magnetischen Fluss, der nur die magnetische Lagerung realisiert.

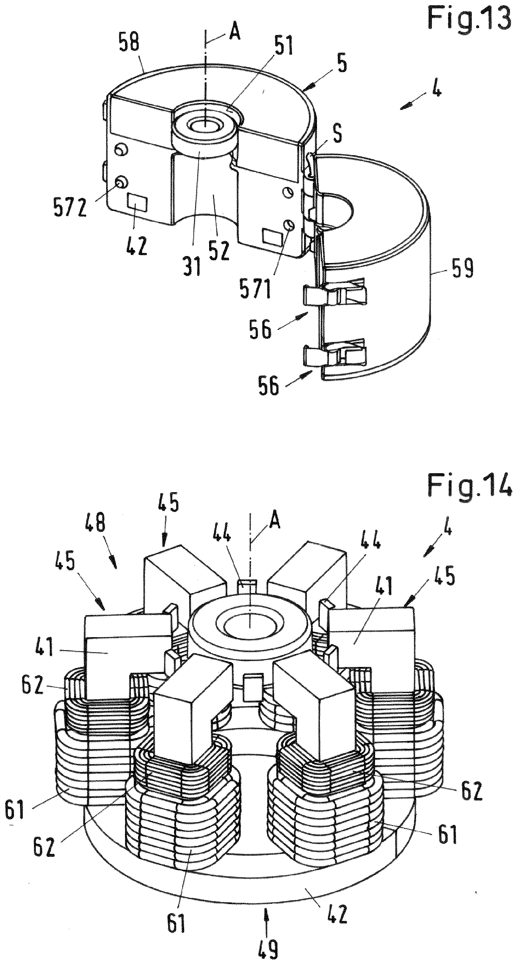

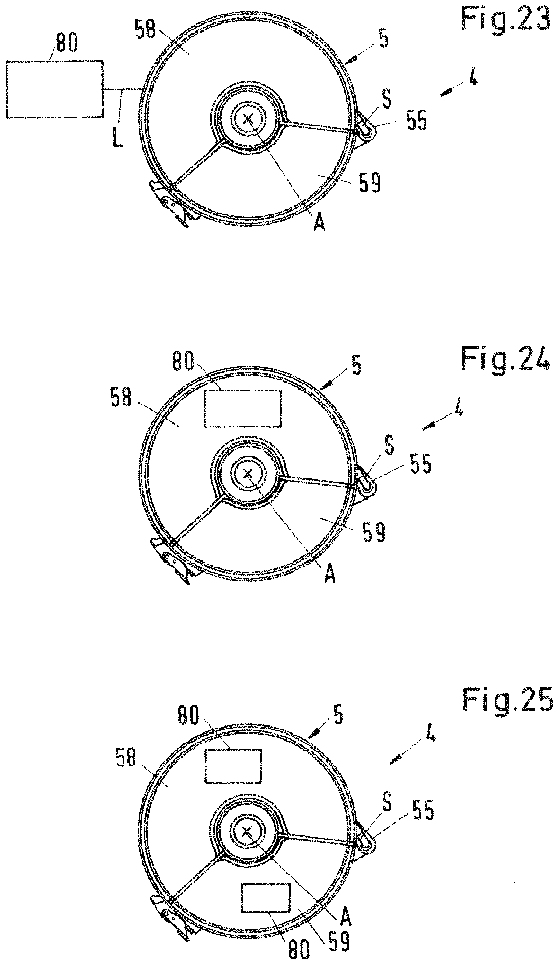

Zur Generierung des Antriebs- und des Steuerfelds ist es einerseits möglich, zwei unterschiedliche Wicklungssysteme zu verwenden (siehe Fig. 14), nämlich eines zur Erzeugung des Antriebsfelds und eines zur Erzeugung des Steuerfelds. Die Spulen zur Erzeugung des Antriebsfelds werden dann üblicherweise als Antriebsspulen bezeichnet und die Spulen zur Erzeugung des Steuerfelds als Steuerspulen. Der Strom, der in diese Spulen eingeprägt wird, wird dann als Antriebsstrom bzw. als Steuerstrom bezeichnet. Andererseits ist es aber auch möglich, die Antriebs- und Lagerfunktion mit nur einem einzigen Wicklungssystem wie in dem hier beschriebenen ersten Ausführungsbeispiel (siehe Fig. 2) zu generieren, sodass es also keine Unterscheidung zwischen Antriebs- und Steuerspulen gibt. Dies kann so realisiert werden, dass die von einer Kontrolleinrichtung 80 (z.B. Fig. 23) jeweils ermittelten Werte für den Antriebs- und den Steuerstrom rechnerisch -also z. B. mit Hilfe von Software - addiert bzw. überlagert werden und der sich daraus ergebende Gesamtstrom in die jeweilige konzentrierte Wicklung 6 eingeprägt wird. In diesem Fall ist es natürlich nicht mehr möglich, zwischen Steuer- und Antriebsspulen zu unterscheiden.

Weitere Details zum Aufbau und zur Ausgestaltung des lagerlosen Motors finden sich beispielsweise in der EP 4 083 431.

Bei dem in Fig. 1 dargestellten ersten Ausführungsbeispiel umfasst die Einmalvorrichtung 100 noch weitere Komponenten, die natürlich auch alle für den Einmalgebrauch ausgestaltet sind, nämlich eine Filtereinrichtung 101, einen Sammelbehälter 102, für ein in der Filtereinrichtung 101 ausgefiltertes Permeat, sowie einen Satz von Verbindungen 103, zum Verbinden von Komponenten. Die Verbindungen 103 können als Schläuche oder als andere Verbindungselemente ausgestaltet sein. Ferner ist ein Bioreaktor 300 zur Kultivierung von Zellen vorgesehen. Dazu ist der Bioreaktor 300 mit einer Zellbrühe (cell broth) gefüllt.

Dabei sind solche Ausgestaltungen möglich, bei welchen der gesamte Bioreaktor 300 als Einmalteil ausgestaltet ist, beispielsweise als ein formstabil ausgestalteter Kunststoffbehälter. Der Bioreaktor 300 ist dann Bestandteil der Einmalvorrichtung 100.

Es sind auch solche Ausgestaltungen möglich, bei denen nur Komponenten des Bioreaktors 300 als Einmalteile ausgestaltet sind. Der Bioreaktor 300 umfasst dann beispielsweise einen flexiblen Einsatz zur Aufnahme des Fluids, welcher aus einem Kunststoff hergestellt ist. Der Einsatz ist vorzugsweise ein flexibler Beutel, beispielsweise ein Plastik- oder ein Kunststoffsack, der zusammengefaltet werden kann, sodass er bei der Lagerung möglichst wenig Platz beansprucht. Der Einsatz kann zusätzliche Ein- oder Auslässe umfassen, beispielsweise für die Zuführung weiterer Substanzen, z. B. Nährlösungen oder Gase wie z. B. Sauerstoff. Auch ist es möglich, einen weiteren Einlass für die Aufnahme von Sonden oder Messsensoren vorzusehen, mit denen Parameter überwacht werden, z. B. Temperatur, Druck, Konzentrationen etc. Der flexible Einsatz ist dann Bestandteil der Einmalvorrichtung. Der Bioreaktor 300 umfasst bei dieser Ausgestaltung ferner einen formstabilen Stützbehälter, der als wiederverwendbare Komponente und für die Aufnahme des Einsatzes ausgestaltet ist. Der Stützbehälter ist dann Bestandteil der wiederverwendbaren Vorrichtung 200.

Ferner sind Ausgestaltungen möglich, bei denen der Bioreaktor 300 gesamthaft als wiederverwendbare Komponente ausgestaltet ist, beispielsweise als ein Edelstahltank, der sterilisierbar ist.

Wie dies in Fig. 1 zu erkennen ist, ist eine Auslassöffnung des Bioreaktors 300 über eine Verbindung 103 mit dem Einlass 21 des als Pumpengehäuse ausgestalteten Rotorgehäuses 2 verbunden, sodass der mit den Flügeln 33 versehene Rotor 3, das Fluid aus dem Bioreaktor 300 fördern kann. Der zweite Auslass 23 des Rotorgehäuses 2 ist mit einem Eingang der Filtereinrichtung 101 verbunden, welche einen Permeatauslass für das Permeat und einen Retentatausgang für das Retentat hat. Der Permeatauslass der Filtereinrichtung ist über eine weitere Verbindung 103 mit dem Sammelbehälter 102 verbunden, in welchem das Permeat gesammelt wird. Der Retentatausgang der Filtereinrichtung ist über eine Verbindung 103 mit einer Einlassöffnung des Bioreaktors 300 verbunden, sodass das Retentat in den Bioreaktor 300 rezirkuliert wird. Der Auslass 22 ist über die Verbindungsleitung 9 mit der Einmalkomponente 10 verbunden, sodass ein Teil des geförderten Fluids aus dem Rotorgehäuse 2 in die Einmalkomponente 10 abgeführt wird.

Mit dem Rotor 3 des elektromagnetischen Drehantriebs wird das Fluid, also beispielsweise die Zellbrühe, durch die Filtereinrichtung 101 gefördert und in den Bioreaktor 300 rezirkuliert, wobei ein Teil des Fluids in die Einmalkomponente 10 abgeführt wird. In der Filtereinrichtung 101 werden beispielsweise Stoffwechselprodukte der Zellen mittels Filtration separiert und als Permeat bzw. Filtrat in den Sammelbehälter 102 abgeführt.

Es versteht sich, dass die Zusammensetzung der Einmalvorrichtung 100 des ersten Ausführungsbeispiels mit beispielhaftem Charakter zu verstehen ist. Je nach Anwendungsfall können auch noch weitere oder andere Komponenten in der Einmalvorrichtung 100 vorgesehen sein.

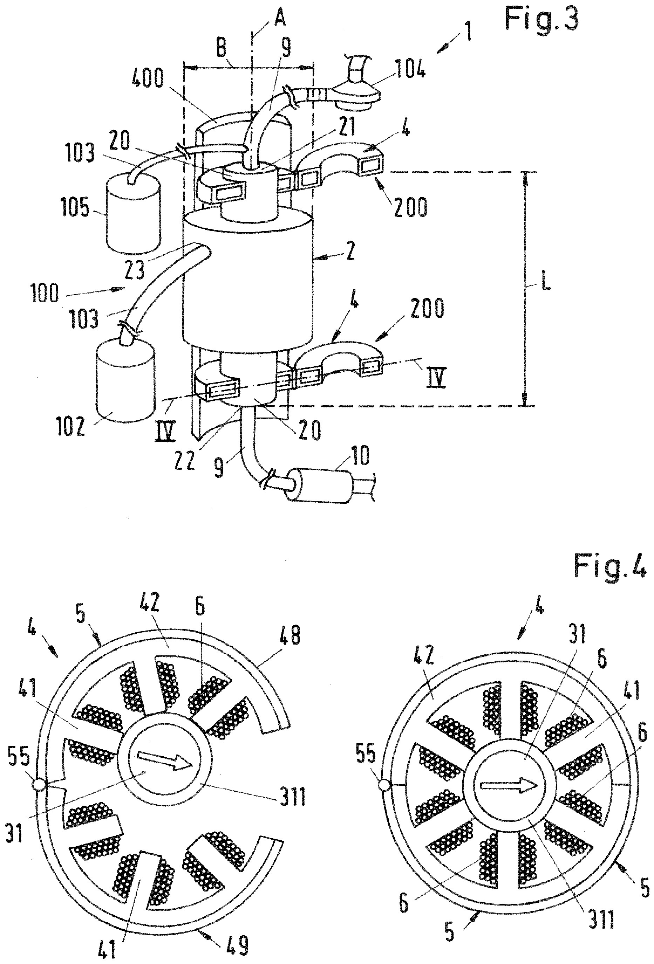

Fig. 3 zeigt in einer perspektivischen Darstellung ein zweites Ausführungsbeispiel eines erfindungsgemässen elektromagnetischen Drehantriebs.

Bei der folgenden Beschreibung des zweiten Ausführungsbeispiels wird nur auf die Unterschiede zu dem ersten Ausführungsbeispiel näher eingegangen. Gleiche Teile oder von der Funktion her gleichwertige Teile des zweiten Ausführungsbeispiels sind mit den gleichen Bezugszeichen bezeichnet wie bei dem ersten Ausführungsbeispiel. Insbesondere haben die Bezugszeichen die gleiche Bedeutung wie sie bereits im Zusammenhang mit dem ersten Ausführungsbeispiel erläutert sind. Es versteht sich, dass alle vorangehenden Erläuterungen des ersten Ausführungsbeispiels in gleicher Weise oder in sinngemäss gleicher Weise auch für das zweite Ausführungsbeispiel gelten.

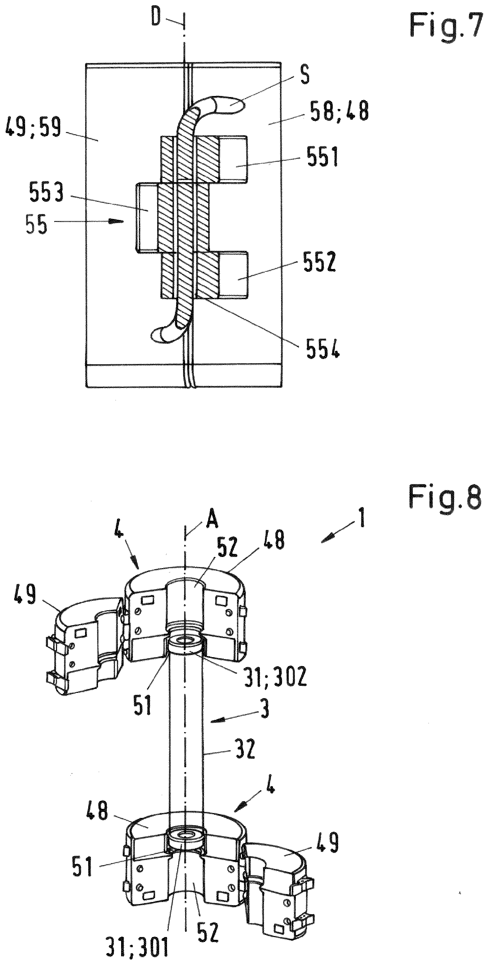

Bei dem zweiten Ausführungsbeispiel des elektromagnetischen Drehantriebs 1 ist der Rotor 3 wiederum für eine berührungslose magnetische Lagerung ausgestaltet. In Fig. 3 ist der Rotor 3 nicht sichtbar, weil er im Inneren des Rotorgehäuses 2 angeordnet ist. Bezüglich der magnetischen Lagerung kann der Rotor 3 jedoch in sinngemäss gleicher Weise ausgestaltet sein, wie das beispielsweise in Fig. 8 dargestellt und weiter hinten beschrieben ist. Im Vergleich zum ersten Ausführungsbeispiel ist der Rotor 3 des zweiten Ausführungsbeispiels mit einer deutlich grösseren Erstreckung bezüglich der axialen Richtung A ausgestaltet. Dementsprechend ist auch das Rotorgehäuse 2 mit einer grösseren axialen Erstreckung ausgestaltet. Die axiale Erstreckung des Rotorgehäuses 2 ist mit dem Bezugszeichen L bezeichnet. Insbesondere ist das Rotorgehäuse 2 mit einer axialen Erstreckung L ausgestaltet, die mindestens so gross und vorzugsweise grösser, beispielsweise mindestens doppelt so gross, ist wie die maximale Erstreckung B des Rotorgehäuses in radialer Richtung. Die maximale Erstreckung B in radialer Richtung ist beispielsweise der maximale Aussendurchmesser des Rotorgehäuses 2.

Bei einer derartig langen Ausgestaltung (bezüglich der axialen Richtung A) des Rotors 3 bzw. des Rotorgehäuses 2, ist es meistens nicht mehr möglich, den Rotor 3 mit nur einer Lagerstelle berührungslos magnetisch zu lagern. Daher sind bei dem zweiten Ausführungsbeispiel zwei Statoren 4 für die magnetische Lagerung des Rotors 3 vorgesehen, wobei mindestens einer der Statoren 4 als Lager- und Antriebsstator ausgestaltet ist, mit welchem die Rotation des Rotors 3 antreibbar ist. Vorzugsweise sind jedoch beide Statoren 4 jeweils als Lager- und Antriebsstator ausgestaltet.

Das Rotorgehäuse 2 umfasst zwei Lagergehäuse 20, welche die beiden axialen Enden des Rotorgehäuses 2 bilden, das heisst am darstellungsgemäss (Fig. 3) oberen axialen Ende des Rotorgehäuses 2 ist ein Lagergehäuse 20 vorgesehen und am darstellungsgemäss unteren axialen Ende des Rotorgehäuses 2 ist ein Lagergehäuse 20 vorgesehen. Vorzugsweise sind die Lagergehäuse 20 integraler Bestandteil des Rotorgehäuses 2.

Jedes Lagergehäuse 20 ist im Wesentlichen zylindrisch ausgestaltet und zentral um die Solldrehachse herum angeordnet. Jedes der Lagergehäuse 20 ist vorzugsweise mit einem Aussendurchmesser ausgestaltet, der kleiner ist als die maximale Erstreckung B des Rotorgehäuses 2 in radialer Richtung. Für jedes Lagergehäuse 20 ist jeweils einer der beiden Statoren 4 vorgesehen, die beide öffenbar ausgestaltet sind, so wie dies anhand des ersten Ausführungsbeispiels erläutert wurde Die beiden Statoren 4 sind an einer Stützstruktur 400 befestigt, welche beispielsweise eine Gehäusewand sein kann. Die beiden Statoren sind bezüglich der axialen Richtung A beabstandet angeordnet, sodass in der Schliessstellung der Statoren 4 einer der beiden Statoren 4 das eine Lagergehäuse 20 umschliesst, und der andere der beiden Statoren 4 das andere Lagergehäuse 20.

Der Rotor 3 erstreckt sich bezüglich der axialen Richtung A von einem ersten Ende 301 (Fig. 8) bis zu einem zweiten Ende 302. An jedem Ende 301, 302 ist jeweils ein magnetisch wirksamer Kern 31 vorgesehen, wobei in jedem Lagergehäuse 20 jeweils einer der magnetisch wirksamen Kerne 31 des Rotors 3 angeordnet ist. Die beiden magnetisch wirksamen Kerne 31 sind mechanisch starr aneinander gekoppelt, so dass beide magnetisch wirksamen Kerne 31 drehfest mit dem Rotor 3 verbunden sind.

Da die Statoren 4 zum Umschliessen der Lagergehäuse 20 des Rotorgehäuses 2 angeordnet und ausgestaltet sind, ist jeder Stator 4 zum Zusammenwirken mit genau einem der beiden magnetisch wirksamen Kerne 31 des Rotors 3 ausgestaltet. In der Schliessstellung der Statoren 4 umgibt jeder Stator 4 jeweils einen der magnetisch wirksamen Kerne 31 entlang des gesamten Umfangs des jeweiligen Lagergehäuses 20.

Wie bereits erwähnt, können die beiden Statoren 4 jeweils als Lager- und Antriebsstator ausgestaltet sein, insbesondere können die beiden Statoren 4 zumindest im Wesentlichen identisch ausgestaltet sein. Je nach Anwendungsfall ist es aber bei dieser Ausführungsform mit zwei Statoren 4, die jeweils als Lager- und Antriebsstator ausgestaltet sind, keinesfalls notwendig, mit beiden Statoren 4 gleichzeitig ein Drehmoment zu erzeugen, welches die Rotation des Rotors 3 antreibt. Es ist beispielsweise auch möglich, nur mit einem der beiden Statoren 4 ein auf den mit ihm zusammenwirkenden magnetisch wirksamen Kern 31 wirkendes Drehmoment zu generieren, und den anderen der beiden Statoren 4 so zu betreiben, dass er kein Drehmoment erzeugt, welches die Rotation des Rotors 3 antreibt.

Prinzipiell sind natürlich auch solche Ausgestaltungen möglich, bei denen einer der beiden Statoren 4 als reiner Lagerstator für die magnetische Lagerung des Rotors 3 ausgestaltet ist. Dieser Lagerstator kann dann nicht zur Drehmomentbildung für das Antreiben des Rotors 3 dienen.

Ein weiterer Unterschied zu dem ersten Ausführungsbeispiel des elektromagnetischen Drehantriebs 1 ist es, dass die beiden jeweils vorzugsweise als Lager- und Antriebsstator ausgestalteten Statoren 4 nicht als Stator 4 eines Tempelmotors, sondern als Stator 4 eines Flachmotors oder Radialmotors ausgestaltet sind. Auch in der Ausgestaltung als Flachmotor wird der elektromagnetische Drehantrieb 1 vorzugsweise nach dem Prinzip des lagerlosen Motors ausgestaltet und betrieben. Mit dem Begriff Flachmotor oder Radialmotor sind dabei solche Ausgestaltungen gemeint, bei denen die Wicklungen 6 zur Erzeugung der elektromagnetischen Drehfelder in der radialen Ebenen angeordnet sind.

Fig. 4 zeigt in einer schematischen Darstellung den Stator4 des zweiten Ausführungsbeispiels in der Offenstellung (links) und in der Schliessstellung (rechts), jeweils in einem Querschnitt senkrecht zur axialen Richtung A entlang der Schnittlinie IV-IV in Fig. 3. Ein entsprechender Schnitt durch den darstellungsgemäss oberen Stator 4 in Fig. 3 sieht gleich aus wie die Darstellung in Fig. 4, weil die beiden Statoren 4 im Wesentlichen identisch ausgestaltet sind. Daher gelten die folgenden Erläuterungen für beide der Statoren 4.

Die magnetisch wirksamen Kerne 31 sind vorzugsweise permanentmagnetisch ausgestaltet. In Fig. 4 ist die Magnetisierung der magnetisch wirksamen Kerne 31 jeweils durch den Pfeil ohne Bezugszeichen dargestellt. Jeder magnetisch wirksame Kern 31 ist vorzugsweise diametral magnetisiert, so wie dies die Pfeile ohne Bezugszeichen in Fig. 4 anzeigen. Jeder magnetisch wirksame Kern 31 ist beispielsweise als permanentmagnetische Scheibe oder als permanentmagnetischer Ring ausgestaltet. Der magnetisch wirksame Kern 31 kann gesamthaft aus einem permanentmagnetischen Material bestehen, oder einen oder mehrere Permanentmagnete umfassen, die beispielsweise in einem weichmagnetischen Material angeordnet sind.

Vorzugsweise ist ferner eine Ummantelung 311 vorgesehen, mit welcher der magnetisch wirksame Kern 31 umschlossen und vorzugsweise hermetisch eingekapselt ist, sodass der magnetisch wirksame Kern 31 nicht in Kontakt mit dem Fluid kommt.

Bei der in Fig. 4 dargestellten Ausgestaltung als Flachmotor umfasst der Stator 4 die Mehrzahl von ausgeprägten Statorpolen 41 - hier sechs Statorpole 41 -, die sich jeweils von einem radial aussenliegenden ringförmigen Rückschluss 42 in radialer Richtung nach innen auf den magnetischen Kern 31 des Rotors 3 hin erstrecken. Jeder Statorpol 41 ist in der radialen Ebene angeordnet und wird jeweils durch eine dem magnetisch wirksamen Kern 31 des Rotors 3 zugewandte Stirnfläche begrenzt. Während des Betriebs des elektromagnetischen Drehantriebs 1 ist es die Soll-Position, dass der magnetisch wirksame Kern 31 zwischen den Stirnflächen der Statorpole 41 zentriert ist.

Um die für den magnetischen Antrieb und die magnetische Lagerung des Rotors 3 notwendigen elektromagnetischen Drehfelder zu erzeugen, tragen die Statorpole 41 die Wicklungen 6, die auch hier wieder als konzentrierte Wicklungen 6 beispielsweise so ausgestaltet sind, dass um jeden Statorpol 41 herum jeweils genau eine konzentrierte Wicklung 6 gewickelt ist, sodass auch jede konzentrierte Wicklung 6 in der radialen Ebene angeordnet ist. Mit diesen konzentrierten Wicklungen 6 werden im Betriebszustand die elektromagnetischen Drehfelder für den Antrieb und die Lagerung des Rotors 3 erzeugt.

Der öffenbar ausgestaltete Stator 4 ist wiederum mit dem ersten Statorteil 48 und dem zweiten Statorteil 49 ausgestaltet, welche über das am Statorgehäuse 5 vorgesehene Gelenk 55 miteinander verbunden sind, sodass der Stator 4 aus der Offenstellung (linke Darstellung in Fig. 4) in die Schliessstellung (rechte Darstellung in Fig. 4) aufgeklappt und umgekehrt aus der Offenstellung in die Schliessstellung zugeklappt werden kann.

Auch in Fig. 3 sind die beiden Statoren 4 jeweils in ihrer Offenstellung dargestellt. Die Darstellung der Statoren 4 in Fig. 3 ist stark schematisiert.

Wie dies insbesondere in Fig. 3 erkennbar ist, sind bei dem zweiten Ausführungsbeispiel zwei Verbindungsschläuche 9 vorgesehen, die jeweils durch einen der Statoren 4 hindurchgeführt werden müssten, wenn die Statoren 4 nicht in die Offenstellung gebracht werden könnten. Auch wäre es bei fixiert angeordneten Statoren 4 nicht möglich, die beiden Lagergehäuse 20 des Rotorgehäuses 2 in die Statoren 4 einzusetzen, weil die maximale Erstreckung B des Rotorgehäuses 2 in radialer Richtung grösser ist als der Aussendurchmesser der Lagergehäuse 20. Ein Einsetzen wäre dann nur möglich, wenn mindestens einer der beiden Statoren in axialer Richtung A verschiebbar angeordnet ist. Aber auch dann wäre ein Zusammensetzen der Einmalvorrichtung 100 erst dann möglich, wenn das Rotorgehäuse bereits in den Statoren angeordnet ist.

Bei der erfindungsgemässen Ausgestaltung kann die bereits vollständig assemblierte Einmalvorrichtung 100 gesamthaft in die Statoren 4 eingesetzt werden. Dies ist in Fig. 3 sehr gut zu erkennen. In der Offenstellung der beiden Statoren 4 kann das Rotorgehäuse 2 mit den beiden daran bereits angeschlossenen Verbindungsschläuchen 9 in einfacher Weise in die beiden Statoren 4 eingesetzt werden. Danach werden die beiden Statoren 4 in die Schliessstellung gebracht, sodass das Rotorgehäuse 2 zwischen den beiden Statoren 4 fixiert ist.

Das zweite Ausführungsbeispiel kann beispielsweise als Separator ausgestaltet sein, der nach dem Prinzip einer Zentrifuge arbeitet und eine fliessfähige Substanz, z.B. eine Suspension in zwei Phasen unterschiedlicher Dichte aufgeteilt. Das Rotorgehäuse 2 mit dem darin angeordneten Rotor 3 ist dann als Separatoreinsatz ausgestaltet, beispielsweise als ein Separatoreinsatz wie er in der bereits zitierten DE 10 2020 121 422 offenbart wird.

Der Einlass 21 des Rotorgehäuses 2 ist über einen der Verbindungsschläuche 9 mit dem Ausgang einer Zentrifugalpumpe dargestellt, von der in Fig. 3 nur ein Pumpengehäuse 104 dargestellt ist, in welchem das Laufrad der Pumpe angeordnet ist. Das Pumpengehäuse 104 kann beispielsweise in sinngemäss gleicher Weise ausgestaltet sein wie das Rotorgehäuse 2 des ersten Ausführungsbeispiels. Die Lager- und Antriebseinheit für das Pumpengehäuse 104 ist in Fig. 3 nicht dargestellt. Die Zentrifugalpumpe mit dem Pumpengehäuse 104 fördert die aufzutrennende Suspension aus einem Vorratsbehälter (nicht dargestellt) zum Einlass 21 des Rotorgehäuses 2. In dem Rotorgehäuse 2 wird die Suspension mittels Zentrifugalkräften in zwei Phasen unterschiedlicher Dichte aufgeteilt. Eine der Phasen wird durch den Auslass 22 in axialer Richtung A durch den anderen Verbindungsschlauch 9 und durch den darstellungsgemäss unteren Stator 4 hindurch in die Einmalkomponente 10 abgeführt, die beispielsweise als Sammelbehälter ausgestaltet ist. Die andere der beiden Phasen wird durch den zweiten Auslass 23, welcher in der Umfangsfläche des Rotorgehäuses 2 angeordnet ist, und durch eine Verbindung 103 in den Sammelbehälter 102 abgeführt.

Optional kann noch ein Reservoir 105 für ein weiters Fluid vorgesehen sein, welches der Suspension zugeführt wird, bevor die Suspension den Einlass 21 des Rotorgehäuses 2 erreicht. Das Reservoir 105 ist hier über eine Verbindung 103 mit dem Verbindungsschlauch 9 verbunden, welcher von dem Pumpengehäuse 104 der Zentrifugalpumpe zum Einlass 21 des Rotorgehäuses 2 führt, wobei die Verbindung 103 stromaufwärts des Einlasses 21 in den Verbindungsschlauch 9 mündet.

Bei dem zweiten Ausführungsbeispiel des elektromagnetischen Drehantriebs 1 umfasst die Einmalvorrichtung 100 also beispielsweise die folgenden Elemente: Das Rotorgehäuse 2 mit dem darin vorgesehenen Rotor 3, die als Sammelbehälter ausgestaltet Einmalkomponente 10, die beiden Verbindungsschläuche 9, das Pumpengehäuse 104 mit dem darin vorgesehenen Laufrad, den Sammelbehälter 102, den Satz von Verbindungen 103 und optional das Reservoir 105.

Die wiederverwendbare Vorrichtung 200 umfasst die beiden Statoren 4 mit den Statorgehäusen 5.

Wie dies am besten in Fig. 3 zu erkennen ist, kann auch bei dem zweiten Ausführungsbeispiel des erfindungsgemässen Drehantriebs 1 die bereits vollständig zusammengesetzte Einmalvorrichtung 100 gesamthaft in die wiederverwendbare Vorrichtung eingesetzt bzw. von dieser getrennt werden, wenn sich die beiden Statoren 4 in der Offenstellung befinden.

Bezugnehmend auf die Fig. 5-7 wird nun eine bevorzugte Ausführungsform des Stators 4 und insbesondere des Statorgehäuses 5 des Stators 4 erläutert, die grundsätzlich für alle im Rahmen dieser Anmeldung erläuterten Statoren 4 geeignet ist. Das Statorgehäuse 5 eignet sich sowohl für Statoren 4 in der Ausgestaltung des elektromagnetischen Drehantriebs 1 als Tempelmotor, als auch für Statoren 4 in der Ausgestaltung des elektromagnetischen Drehantriebs 1 als Flachmotor oder Radialmotor.

Fig. 5 und Fig. 6 zeigen jeweils eine perspektivische Darstellung des Stators 4, wobei in Fig. 5 und Fig. 6 jeweils nur das Statorgehäuse 5 des Stators 4 zu erkennen ist, weil die innerhalb des Statorgehäuses 5 angeordneten Komponenten des Stators, wie beispielsweise die Statorpole 41, durch das Statorgehäuse 5 verdeckt sind. Zum besseren Verständnis ist in Fig. 5 und in Fig. 6 jeweils der magnetisch wirksame Kern 31 des Rotors 3 dargestellt, mit welchem der Stator 4 zusammenwirkt. Fig. 5 zeigt den Stator 4 in der Schliessstellung, und Fig. 6 zeigt den Stator 4 in der Offenstellung.

Fig. 7 zeigt in einer Schnittdarstellung das Gelenk 55, welches die beiden Statorteile 48, 49 miteinander verbindet, in einem Schnitt in axialer Richtung A.

Das Statorgehäuse 5 umfasst das erste Gehäuseteil 58, welche die Ummantelung des ersten Statorteils 48 bildet, sowie das zweite Gehäuseteil 59, welches die Ummantelung des zweiten Statorteils 49 bildet. Vorzugsweise ist der erste Statorteil 48 durch das erste Gehäuseteil 58 gekapselt, und der zweite Statorteil 49 ist durch das zweite Gehäuseteil 59 gekapselt. Vorzugsweise ist die Kapselung des ersten Statorteils 48 und die Kapselung des zweiten Statorteils 59 jeweils als hermetische Kapselung ausgestaltet.

Das Statorgehäuse 5 hat eine im Wesentlichen zylindrische Ausgestaltung und wird in axialer Richtung A von zwei Stirnflächen begrenzt. In einer der beiden Stirnflächen ist die zentrale Ausnehmung 51 vorgesehen, in welche das Rotorgehäuse 2 einsetzbar ist. Von der Ausnehmung 51 erstreckt sich die Durchführung 52, vorzugsweise in axialer Richtung A und im Zentrum des Statorgehäuses 5 bis zu der anderen Stirnfläche des Statorgehäuses 5.

Jedes der beiden Gehäuseteile 58, 59 ist vorzugsweise jeweils als separates Gehäuseteil 58, 59 ausgestaltet, welche gemeinsam das zylindrische Gehäuse 5 bilden. Somit ist jedes Gehäuseteil 58, 59 jeweils als Zylindersegment ausgestaltet, wobei sich die Zylindersegmente zu einem Zylinder ergänzen.

In dem ersten Gehäuseteil 58 sind die Spulenkerne 45 und/oder die Statorpole 41 sowie die Wicklungen 6 angeordnet, welche zu dem ersten Statorteil 48 gehören, sowie der Teil des Rückschlusses 42, der diese Spulenkerne 45 miteinander verbindet.

In dem zweiten Gehäuseteil 59 sind die Spulenkerne 45 und/oder die Statorpole 41 sowie die Wicklungen 6 angeordnet, welche zu dem zweiten Statorteil 48 gehören, sowie der Teil des Rückschlusses 42, der diese Spulenkerne 45 miteinander verbindet.

Die beiden Gehäuseteile 58, 59 können in Umfangsrichtung gesehen unterschiedlich lang ausgestaltet sein. Wie dies insbesondere in Fig. 5 zu erkennen ist, ist bei der hier beschriebenen Ausführungsform das erste Gehäuseteil 58 in Umfangsrichtung gesehen länger ausgestaltet als das zweite Gehäuseteil 59. Diese Ausführungsform ist insbesondere dann bevorzugt, wenn der Stator 4 eine ungerade Anzahl von Statorpolen 41 oder Spulenkernen 45 aufweist, beispielsweise fünf Statorpole 41 oder fünf Spulenkerne 45 (siehe z.B. Fig. 11 und Fig. 12). Dann sind in dem ersten Gehäuseteil 58 beispielsweise drei Statorpole 41 oder drei Spulenkerne 45 vorgesehen und in dem zweiten Gehäuseteil 59 zwei Statorpole 41 oder zwei Spulenkerne 45.

Die Kontrolleinrichtung 80, welche beispielsweise die Leistungselektronik für die Ansteuerung und die Versorgung der Wicklungen 6 sowie Signalverarbeitungselektronik umfasst, kann in einem der beiden Gehäuseteile 58, 59 angeordnet sein, oder sie kann auf das erste Gehäuseteil 58 und das zweite Gehäuseteil 59 verteilt werden, wie weiter hinten noch beschrieben wird.

In der Schliessstellung (siehe Fig. 5) grenzen die beiden jeweils als Zylindersegmente ausgestalteten Gehäuseteile 58, 59 an zwei Grenzflächen aneinander. An einer dieser Grenzflächen ist das Gelenk 55 angeordnet, welches die beiden Gehäuseteile 58, 59 gelenkig miteinander verbindet. Die andere Grenzfläche wird als Stossstelle 589 bezeichnet. In der Schliessstellung des Stators 4 stossen die beiden Gehäuseteile 58, 59 an der Stossstelle 589 aneinander an. In der Offenstellung des Stators 4 ist der Stator 4 in Umfangsrichtung gesehen an der Stossstelle geöffnet (Fig. 6), sodass sich die beiden Gehäuseteile 58, 59 hier nicht berühren.

Das erste Gehäuseteil 58 und das zweite Gehäuseteil 59 sind über das Gelenk 55 miteinander verbunden, sodass die beiden Gehäuseteile 58, 59 relativ zueinander schwenkbar sind. Das Gelenk 55 ist an der äusseren Umfangsfläche des Statorgehäuses 5 angeordnet. Durch eine Bewegung des zweiten Gehäuseteils 59 relativ zum ersten Gehäuseteil 58 kann der Stator 4 aufgeklappt werden, d.h. von der Schliessstellung (Fig. 5) in die Offenstellung (Fig. 6) gebracht werden, und zugeklappt werden, d.h. von der Offenstellung in die Schliessstellung gebracht werden. Die Schwenkbewegung, mit welcher das zweite Gehäuseteil 59 relativ zum ersten Gehäuseteil 58 bewegt werden kann, erfolgt um eine Achse, die sich in axialer Richtung A erstreckt.

Wie bereits erwähnt ist jedes Gehäuseteil 58, 59 vorzugsweise als eine Kapselung, insbesondere als eine hermetische Kapselung, des jeweiligen Statorteils 48, 49 ausgestaltet, d.h. jeder Statorteil 48, 49 ist vorzugsweise jeweils für sich gekapselt ausgestaltet.

Die zweiteilige Ausgestaltung des Stators 4 hat zur Folge, dass der magnetische Rückschluss 42, welcher die Statorpole 41 magnetisch miteinander koppelt, an zwei Stellen unterbrochen bzw. aufgetrennt ist, nämlich an den beiden Grenzflächen zwischen den beiden Gehäuseteilen 58, 59. Diese Unterbrechungen werden so ausgestaltet, dass sie eine möglichst geringe Störung des magnetischen Flusses in dem Rückschluss 42 darstellen. Dies kann beispielsweise durch eine besonders dünne Ausgestaltung des jeweiligen Gehäuseteils 58, 59 an den Grenzflächen und im Bereich des Rückschlusses 42 erreicht werden, wie dies durch die Rechtecke in den Grenzflächen in Fig. 6 angedeutet ist. Auch ist es möglich, an diesen Unterbrechungen im Rückschluss 42 diesen mit einer Oberflächenbeschichtung auszugestalten, welche dichtend mit den jeweiligen Gehäuseteilen 58 bzw. 59 verbunden ist, sodass die Einkapselung des ersten Statorteils 48 und des zweiten Statorteils 49 gewährleistet ist.

Durch solche Massnahmen ist es möglich, beide Statorteile 48, 49 jeweils gekapselt auszugestalten und gleichzeitige eine gute magnetische Verbindung zwischen den beiden Statorteilen 48, 49 über den Rückschluss 42 zu gewährleisten.

Ferner ist es bevorzugt, wenn am Statorgehäuse 5 eine mechanische Fixierung 56 vorgesehen ist, mit welcher in der Schliessstellung des Stators 4 (Fig. 5) der erste Statorteil 48 lösbar am zweiten Statorteil 49 fixiert ist, um ein unbeabsichtigtes Aufklappen des Stators 4 sicher zu verhindern. Bei der hier beschriebenen Ausführungsform umfasst die mechanische Fixierung 56 zwei Spannverschlüsse, welche jeweils die Stossstelle 589 zwischen den beiden Gehäuseteilen 58, 59 übergreifen. Die beiden Spannverschlüsse sind an der äusseren Umfangsfläche des Statorgehäuses 5 und bezüglich der axialen Richtung A zueinander beabstandet angeordnet. Jeder Spannverschluss kann beispielsweise in an sich bekannter Weise wie folgt ausgestaltet sein. An einem der beiden Gehäuseteile 58, 59 - hier an dem zweiten Gehäuseteil 59 - ist eine Klaue 561 an einer klappbaren Lasche 562 befestigt, welche an dem zweiten Gehäuseteil 59 fixiert ist. Die klappbare Lasche 562 dient zum Spannen der Klaue 561. An dem anderen Gehäuseteil, hier also dem ersten Gehäuseteil 58 ist ein Vorsprung 563 befestigt, welcher von der Klaue 561 übergreifbar ist. Nachdem der Stator 4 aus der Offenstellung in die Schliessstellung gebracht worden ist, wird die Klaue 561 über den Vorsprung 563 gelegt und mittels der klappbaren Lasche 562 verspannt. Dadurch ist das erste Gehäuseteil 58 am zweiten Gehäuseteil 59 fixiert und der Stator 4 gegen ein unbeabsichtigtes Öffnen gesichert. Zum Öffnen des Stators 4 wird durch Betätigen der Lasche 562 die Klaue 561 von dem Vorsprung 563 gelöst, und der Stator 4 kann aus der Schliessstellung in die Offenstellung aufgeklappt werden.

Ferner ist es bevorzugt, dass mindestens eine mechanische Zentrierung vorgesehen ist, welche in der Schliessstellung des Stators 4 den ersten Statorteil 48 relativ zum zweiten Statorteil 49 zentriert. Als mechanische Zentrierung sind hier Ausnehmungen 571 und Erhebungen 572 vorgesehen, die so ausgestaltet und angeordnet sind, dass jede Erhebung 572 in der Schliessstellung des Stators 4 jeweils in eine der Ausnehmungen 571 eingreift, vorzugsweise passgenau eingreift, und damit die beiden Statorteile 48, 49 relativ zueinander zentriert. Beispielsweise sind zwei Erhebungen 572 und zwei Ausnehmungen 571 in den Oberflächen der Gehäuseteile 58, 59 vorgesehen, welche in der Schliessstellung die Stossstelle 589 bilden, wobei die Erhebungen 572 in der Oberfläche des ersten Gehäuseteils 58 vorgesehen sind und die Ausnehmungen 571 in der Oberfläche des zweiten Gehäuseteils 59. Ferner ist es möglich, auch in der anderen Grenzfläche zwischen den beiden Gehäuseteilen 58, 59, also dort, wo das Gelenk 55 vorgesehen ist, Ausnehmungen 571 und in diese eingreifende Erhebungen 572 als Zentrierung vorzusehen. Die mechanischen Zentrierungen 571, 572 gewährleisten ein sich vollständiges Überdecken des magnetischen Rückschlusses 42 an den beiden Unterbrechungen in den Grenzflächen, so dass eine möglichst geringe Störung des magnetischen Flusses an diesen Unterbrechungen gewährleistet ist. Versätze im magnetischen Kreis lassen sich durch die mechanischen Zentrierungen 571, 572 effizient verhindern.

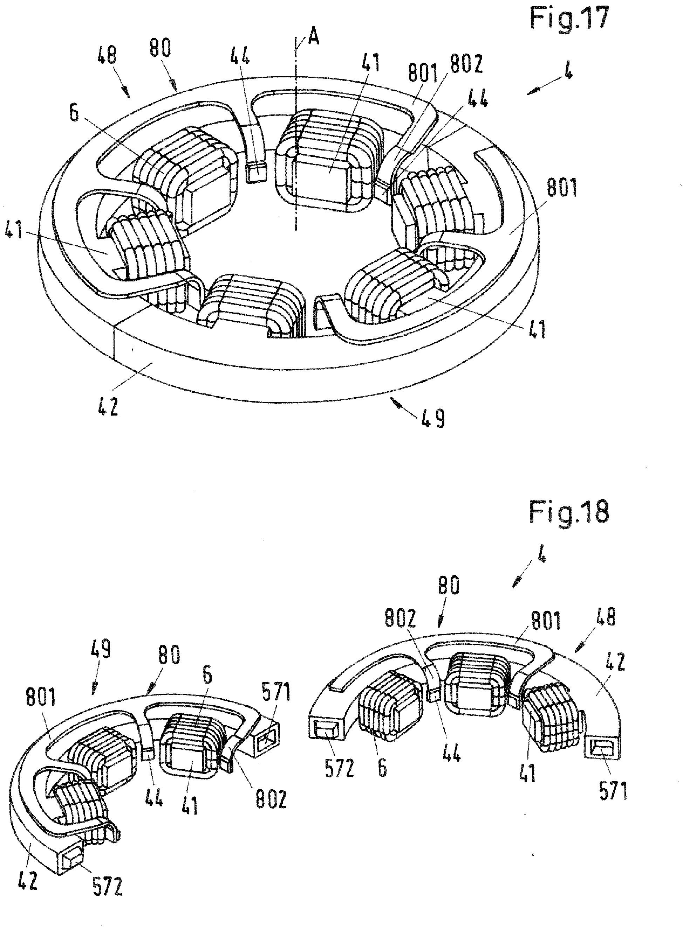

Alternativ oder ergänzend ist es auch möglich, die Erhebungen 572 und die Ausnehmungen 571 im magnetischen Rückschluss 42 vorzusehen (siehe z. B. Fig. 18).

Wie dies in Fig. 7 dargestellt ist, besteht eine weitere bevorzugte Massnahme darin, zwischen dem ersten Statorteil 48 und dem zweiten Statorteil 49 eine elektrische Verbindung S vorzusehen, welche durch das Gelenk 55 hindurchgeführt ist.

In Fig. 7 ist das Gelenk 55 in einer Schnittdarstellung gezeigt. Das Gelenk 55 ist so ausgestaltet, dass der zweite Statorteil 49 relativ zum ersten Statorteil 48 schwenkbar ist, um den Stator 4 aus der Schliessstellung in die Offenstellung zu bringen oder aus der Offenstellung in die Schliessstellung. Die Schwenkbewegung erfolgt um eine Drehachse D, die sich in axialer Richtung A erstreckt. Das Gelenk 55 umfasst ein erstes Gelenkteil 551 und ein zweites Gelenkteil 552, die beide an dem ersten Gehäuseteil 58 fixiert und drehfest mit diesem verbunden sind. Das erste Gelenkteil 551 und das zweite Gelenkteil 552 sind bezüglich der axialen Richtung A beabstandet voneinander angeordnet. Ein drittes Gelenkteil 553 ist an dem zweiten Gehäuseteil 59 fixiert und drehfest mit diesem verbunden. Das dritte Gelenkteil 553 ist bezüglich der axialen Richtung A zwischen dem ersten Gelenkteil 551 und dem zweiten Gelenkteil 552 angeordnet und drehbar relativ zu dem ersten und dem zweiten Gelenkteil 551, 552 gelagert, sodass das dritte Gelenkteil 553 relativ zu dem ersten und dem zweiten Gelenkteil 551, 552 um die Drehachse D schwenkbar bzw. drehbar ist. Jedes der Gelenkteile 551, 552, 553 weist jeweils eine zentrale sich in axialer Richtung A durch das gesamte Gelenkteil 551, 552, 553 erstreckende Passage auf, wobei diese drei Passagen miteinander fluchtend ausgestaltet sind, sodass sich insgesamt eine zentrale Passage 554 ergibt, welche sich in axialer Richtung A durch das gesamte Gelenk 55 hindurch erstreckt. In dieser Passage 554 ist die elektrische Verbindung S angeordnet, über welche der erste Statorteil 48 elektrisch mit dem zweiten Statorteil 49 verbunden ist.

Die elektrische Verbindung S zwischen dem ersten Statorteil 48 und dem zweiten Statorteil 49 zentral in axialer Richtung A durch das Gelenk 55 hindurchzuführen, also in der Drehachse D des Gelenks 55, hat den Vorteil, dass es bei den relativen Schwenkbewegungen zwischen den beiden Statorteilen 48, 49 höchstens zu sehr geringen Verformungen der elektrischen Verbindung S kommt, was besonders materialschonend und vorteilhaft im Hinblick auf eine lange Lebensdauer ist.

Zum besseren Verständnis ist in den Fig. 5-7 die elektrische Verbindung S ausserhalb des Gelenks 55 als offenliegende Leitung dargestellt. Für viele Anwendungen ist es jedoch bevorzugt, wenn die elektrische Verbindung S nicht offenliegend ist, sondern beispielsweise durch eine Abdeckung verdeckt ist.

Natürlich sind auch andere Ausgestaltungen und Anordnungen der elektrischen Verbindung S zwischen dem ersten Statorteil 48 und dem zweiten Statorteil 49 möglich, beispielsweise elektrische Verbindungen, die ausserhalb der Drehachse D angeordnet sind, oder flexible Ausgestaltungen mittels flexiblen elektrischen Verbindungen, die für die Verbindung zwischen relativ zueinander bewegbaren, insbesondere schwenkbaren Komponenten ausgestaltet sind.

Im Hinblick auf die bevorzugten Materialien für das Statorgehäuse 5 ist es bevorzugt, dass das Statorgehäuse 5 zumindest in den Bereichen, wo der Rückschluss 42 aufgetrennt ist und durch die Gehäuseteile 58, 59 geschützt wird, nämlich an den beiden Grenzflächen zwischen den beiden Gehäuseteilen 58, 59, aus Materialien besteht, die elektrisch nicht leitfähig oder zumindest nur schwach leitfähig und trotzdem strapazierfähig sind, beispielsweise im Hinblick auf mechanische Beanspruchungen. Zudem sollte das Statorgehäuse 5 aus Materialien gefertigt sei, die lösungsmittelbeständig sind, so dass das Statorgehäuse 5 mit Alkohol oder anderen Lösungsmitteln gereinigt werden kann. Zu den nicht leitfähigen Materialien gehören beispielsweise Kunststoffe wie Polyetheretherketon (PEEK) oder Polyoxymethylen Copolymer (POM-C). Zu den relativ schlecht leitfähigen Materialien gehören Metalllegierungen wie beispielsweise Hastelloy oder hochlegierte Titan Legierungen (beispielsweise Grade 5).

Ferner ist es eine bevorzugte Massnahme, dass jedes Gehäuseteil 58, 59 mit den darin jeweils angeordneten Komponenten des Stators 4 vorzugsweise mit einem Kunststoff ausgegossen wird, beispielsweise mit einem Epoxidharz, Polyuretan oder Silikon. Das hat den Vorteil, dass der Stator 4 sehr formstabil bleibt, selbst wenn das Statorgehäuse 5 dünnwandig ausgestaltet ist.

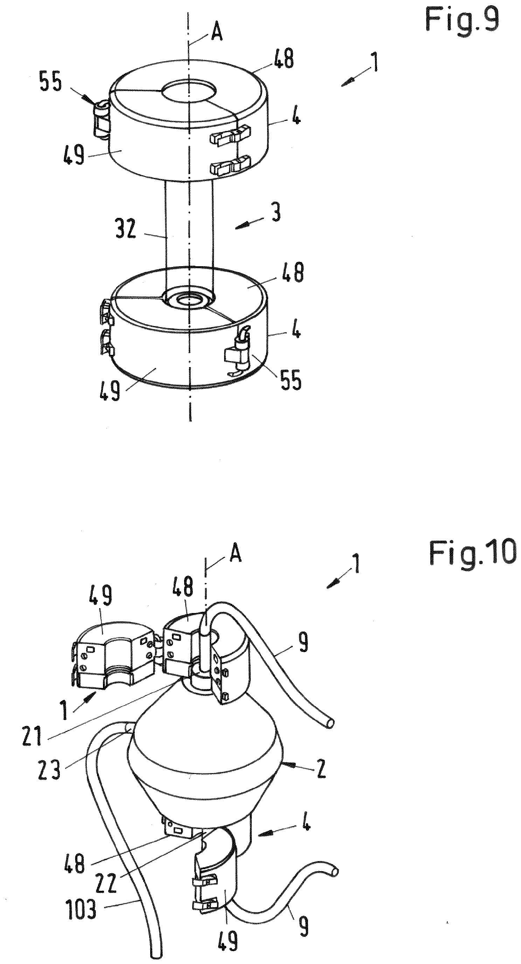

Fig. 8 zeigt eine perspektivische Darstellung eines dritten Ausführungsbeispiels eines erfindungsgemässen elektromagnetischen Drehantriebs 1, wobei der Stator 4 in der Offenstellung ist. Fig. 9 zeigt dieses dritte Ausführungsbeispiel mit dem Stator 4 in der Schliessstellung. Zum besseren Verständnis ist in den Fig. 8 und Fig. 9 das Rotorgehäuse 2 nicht dargestellt. Es versteht sich, dass auch bei dem dritten Ausführungsbeispiel der Rotor 3 in einem Rotorgehäuse 2 angeordnet ist, das beispielsweise analog zu dem zweiten Ausführungsbeispiel ausgestaltet sein kann. Auch sind in den Fig. 8 und Fig. 9 der Verbindungsschlauch 9 oder die Verbindungsschläuche 9 nicht dargestellt, weil diese am Rotorgehäuse 3 angeordnet sind.

Bei der folgenden Beschreibung des dritten Ausführungsbeispiels wird nur auf die Unterschiede zu dem ersten und dem zweiten Ausführungsbeispiel näher eingegangen. Gleiche Teile oder von der Funktion her gleichwertige Teile des dritten Ausführungsbeispiels sind mit den gleichen Bezugszeichen bezeichnet wie bei dem ersten und dem zweiten Ausführungsbeispiel. Insbesondere haben die Bezugszeichen die gleiche Bedeutung wie sie bereits im Zusammenhang mit dem ersten bzw. dem zweiten Ausführungsbeispiel erläutert sind. Es versteht sich, dass alle vorangehenden Erläuterungen in gleicher Weise oder in sinngemäss gleicher Weise auch für das dritte Ausführungsbeispiel gelten.

Bei dem dritten Ausführungsbeispiel ist der Rotor 3 ebenfalls für eine berührungslose magnetische Lagerung ausgestaltet. Ähnlich wie bei dem zweiten Ausführungsbeispiel ist der Rotor 3 mit einer grossen Erstreckung in axialer Richtung A ausgestaltet. Bei einer dermassen langen Ausgestaltung des Rotors 3 bezüglich der axialen Richtung A ist es meistens nicht mehr möglich, den Rotor 3 mit nur einer einzigen Lagerstelle zuverlässig berührungslos magnetisch bezüglich des Stators 4 zu lagern. Daher sind in sinngemäss gleicher Weise wie bei dem zweiten Ausführungsbeispiel bei dem dritten Ausführungsbeispiels zwei Statoren 4 für die magnetische Lagerung des Rotors 3 vorgesehen.

Der Rotor 3 erstreckt sich in axialer Richtung A von dem ersten Ende 301 bis zu dem zweiten Ende 302, wobei an jedem Ende 301, 302 ein magnetisch wirksamer Kern 31 vorgesehen ist. Es sind zwei Statoren 4 vorgesehen, die bezüglich der axialen Richtung A beabstandet angeordnet sind, und von denen jeder öffenbar ausgestaltet ist. Jeder Stator 4 ist zum Zusammenwirken mit genau einem der magnetisch wirksamen Kerne 31 angeordnet und ausgestaltet, derart dass der Rotor 3 im Betriebszustand berührungslos magnetisch bezüglich der Statoren 4 lagerbar ist.

Von dem Rotor 3 ist neben den beiden magnetisch wirksamen Kernen 31, die an den beiden Enden 301, 302 angeordnet sind, nur ein Stab 32 dargestellt, welcher sich von dem magnetisch wirksamen Kern 31 am ersten Ende 301 des Rotors 3 in axialer Richtung A bis zu dem magnetisch wirksamen Kern 31 am zweiten Ende 302 des Rotors 3 erstreckt. Der Stab 32 ist beispielsweise aus Kunststoff gefertigt. Jeder magnetisch wirksame Kern 31 ist drehfest mit dem Stab 32 verbunden. Der Stab 32 bildet eine mechanisch starre Kopplung zwischen den beiden magnetisch wirksamen Kernen 31 an den Enden 301, 302 des Rotors 3, sodass die beiden magnetisch wirksamen Kerne 31 nicht relativ zueinander bewegt werden können.

Je nach Anwendungsfall umfasst der Rotor 3 noch weitere Komponenten, welche in Fig. 8 und Fig. 9 nicht dargestellt sind. Ist der elektromagnetische Drehantrieb 1 beispielsweise als Mischer ausgestaltet, so können an dem Stab 32 ein oder mehrere Flügelräder zur Durchmischung von mindestens zwei Substanzen vorgesehen sein. Der elektromagnetische Drehantrieb 1 kann auch als Rotationsfilter ausgestaltet sein. Dann umfasst der Rotor 3 mindestens ein Filterelement. Der elektromagnetische Drehantrieb 1 kann auch als Separator zur Auftrennung einer fliessfähigen Substanz in verschiedene Phasen mittels Zentrifugalkräften ausgestaltet sein (siehe auch das vierte Ausführungsbeispiel). Dann umfasst der Rotor 3 beispielsweise eine Mehrzahl von Trenntellern, die drehfest mit dem Stab 32 verbunden sind.

Jeder der beiden Statoren 4 ist insbesondere so ausgestaltet, wie dies im Zusammenhang mit den Fig. 5 und Fig. 6 erläutert ist, also jeweils mit dem Statorgehäuse 5, das die beiden Gehäuseteile 58, 59 umfasst, die mittels des Gelenks 55 miteinander verbunden sind.

Die beiden Statoren 4 sind im Wesentlichen identisch ausgestaltet und insbesondere so, wie es anhand der Fig. 5 und Fig. 6 erläutert wurde. Die beiden Statoren 4 sind so angeordnet, dass ihre axialen Mittelachsen miteinander fluchten und auf der Solldrehachse liegen. Ferner sind die Statoren 4 so angeordnet, dass sich die beiden zentralen Ausnehmungen 51 für den Rotor 3 bzw. das Rotorgehäuse 2 gegenüberliegen. Der darstellungsgemäss untere Stator 4 ist so angeordnet, dass die zentrale Ausnehmung 51 in der darstellungsgemäss oberen Stirnfläche dieses Stators 4 ist, und der darstellungsgemäss obere Stator 4 ist so angeordnet, dass seine zentrale Ausnehmung 51 in der darstellungsgemäss unteren Stirnfläche dieses Stators 4 ist.

Wenn der Rotor 3 bzw. das Rotorgehäuse 2 in die Statoren 4 eingesetzt ist, dann ist der magnetisch wirksame Kern 31 am ersten Ende 301 des Rotors 3 in der zentralen Ausnehmung 51 des darstellungsgemäss unteren Stators 4 angeordnet, und der magnetisch wirksame Kern 31 am zweiten Ende 302 des Rotors ist in der zentralen Ausnehmung 51 des darstellungsgemäss oberen Stators 4 angeordnet. Der mindestens eine Verbindungsschlauch 9 (in Fig. 8 und Fig. 9 nicht dargestellt) ist in der Durchführung 52 des oberen oder des unteren Stators 4 angeordnet. Natürlich kann auch bei jedem Stator 4 in der Durchführung 52 jeweils ein Verbindungsschlauch 9 vorgesehen sein.

Auch bei dem dritten Ausführungsbeispiel kann die bereits vollständig assemblierte Einmalvorrichtung 100, von welcher in den Fig. 8 und Fig. 9 nur der Rotor 3 dargestellt ist, gesamthaft in die beiden Statoren 4 eingesetzt werden, wenn die beiden Statoren in der in Fig. 9 dargestellten Offenstellung sind. Auch ist es in der Offenstellung der Statoren 4 möglich, die Einmalvorrichtung 100 gesamthaft aus den beiden Statoren 4 herauszunehmen, ohne dass die Einmalvorrichtung 100 vorher in ihre Bestandteile zerlegt werden muss oder Komponenten der Einmalvorrichtung 100 voneinander getrennt werden müssten.

Auch bei dem dritten Ausführungsbeispiel umfasst die Einmalvorrichtung 100 mindestens die folgenden Komponenten: das Rotorgehäuse 2 mit dem darin angeordneten Rotor 3, mindestens eine Einmalkomponente 10 (beispielsweise einen Vorratsbehälter) mindestens einen Verbindungsschlauch 9, welcher das Rotorgehäuse 3 mit der Einmalkomponente 10 verbindet.

Wie bereits erwähnt ist es nicht notwendig, dass die beiden Statoren 4 gleich ausgestaltet sind. So ist es beispielsweise möglich, wie das auch schon für das zweite Ausführungsbeispiel erläutert wurde, dass einer der beiden Statoren 4 als reiner Lagerstator für die magnetische Lagerung des Rotors 3 ausgestaltet ist, sodass mit diesem Stator 4 kein Drehmoment auf den Rotor ausgeübt 3 werden kann.

Gemäss einer anderen Variante des dritten Ausführungsbeispiels ist nur einer der beiden Statoren öffenbar ausgestaltet, während der andere Stator klassisch ausgestaltet ist, also nicht in eine Offenstellung gebracht werden kann. Das Rotorgehäuse 2 kann dann in axialer Richtung A in diesen nicht öffenbar ausgestalteten Stator eingesetzt werden, während es in den anderen Stator 4, der öffenbar ausgestaltet ist, von der Seite, also in radialer Richtung, eingesetzt wird.

Fig. 10 zeigt eine perspektivische Darstellung eines vierten Ausführungsbeispiels eines erfindungsgemässen elektromagnetischen Drehantriebs 1, wobei der Stator 4 in der Offenstellung ist. In Fig. 10 ist der Rotor 3 nicht sichtbar, weil er im Inneren des Rotorgehäuses 2 angeordnet ist. Bezüglich der magnetischen Lagerung kann der Rotor 3 jedoch in sinngemäss gleicher Weise ausgestaltet sein, wie das für das dritte Ausführungsbeispiel beschrieben wurde.

Bei der folgenden Beschreibung des vierten Ausführungsbeispiels wird nur auf die Unterschiede zu dem ersten, zweiten und dritten Ausführungsbeispiel näher eingegangen. Gleiche Teile oder von der Funktion her gleichwertige Teile des vierten Ausführungsbeispiels sind mit den gleichen Bezugszeichen bezeichnet wie bei dem ersten, zweiten und dritten Ausführungsbeispiel. Insbesondere haben die Bezugszeichen die gleiche Bedeutung wie sie bereits im Zusammenhang mit dem ersten bzw. dem zweiten bzw. dem dritten Ausführungsbeispiel erläutert sind. Es versteht sich, dass alle vorangehenden Erläuterungen in gleicher Weise oder in sinngemäss gleicher Weise auch für das vierte Ausführungsbeispiel gelten.

Das vierte Ausführungsbeispiel stellt eine spezielle Ausgestaltung des dritten Ausführungsbeispiels dar, bei welcher das Rotorgehäuse 2 mit dem darin angeordneten Rotor 3 als Separator ausgestaltet ist, der nach dem Prinzip einer Zentrifuge arbeitet und eine fliessfähige Substanz, z.B. eine Suspension, in zwei Phasen unterschiedlicher Dichte aufgeteilt. Das Rotorgehäuse 2 mit dem darin angeordneten Rotor 3 ist dann als Separatoreinsatz ausgestaltet, beispielsweise als ein Separatoreinsatz wie er in der bereits zitierten DE 10 2020 121 422 offenbart wird.

Bei dem vierten Ausführungsbeispiel umfasst die Einmalvorrichtung 100 zwei Verbindungsschläuche 9

Der Einlass 21 des Rotorgehäuses 2 ist über einen der Verbindungsschläuche 9 mit einer Quelle für die Suspension verbunden, beispielsweise eine Pumpvorrichtung (nicht dargestellt), welche die Suspension aus einem Vorratsbehälter (nicht dargestellt), durch den Verbindungsschlauch 9 zum Einlass 21 des Rotorgehäuses 2 fördert. In dem Rotorgehäuse 2 wird die Suspension mittels Zentrifugalkräften in zwei Phasen unterschiedlicher Dichte aufgeteilt. Eine der Phasen, nämlich die leichtere Phase (Phase mit geringerer Dichte), wird durch den Auslass 22 in axialer Richtung A durch den anderen Verbindungsschlauch 9 und durch den darstellungsgemäss unteren Stator 4 hindurch in die Einmalkomponente 10 (nicht dargestellt) abgeführt, wobei die Einmalkomponente 10 beispielsweise als Sammelbehälter ausgestaltet ist. Die andere der beiden Phasen, nämlich die schwerere Phase (Phase mit höherer Dichte), wird durch den zweiten Auslass 23, welcher an der radial äusseren Begrenzungsfläche des Rotorgehäuses 2 angeordnet ist, und durch eine Verbindung 103 in einen Sammelbehälter 102 (nicht dargestellt) abgeführt.

Wenn beide Statoren 4, wie in Fig. 10 dargestellt, in der Offenstellung sind, kann das Rotorgehäuse 2 mit dem darin angeordneten Rotor 3 und mit den bereits mit dem Rotorgehäuse 2 verbundenen Verbindungsleitungen 9 und mit der mit einer der Verbindungsleitungen 9 verbundenen Einmalkomponente 10 (nicht dargestellt) in radialer Richtung in die beiden Statoren 4 eingesetzt werden. Auch bei dem vierten Ausführungsbeispiel kann also die bereits vollständig assemblierte Einmalvorrichtung 100 in die Statoren 4 eingesetzt werden, also mit der wiederverwendbaren Vorrichtung 200 zusammengefügt, bzw. von dieser getrennt werden.

Wenn die Einmalvorrichtung 100 in die beiden Statoren 4 eingesetzt ist, werden die beiden Statoren 4 durch Zuklappen in die Schliessstellung gebracht und mittels der mechanischen Fixierungen 56 in der Schliessstellung gegen ein ungewolltes Ausklappen gesichert. Somit ist das Rotorgehäuse 2 sicher zwischen den beiden Statoren 4 fixiert. Wenn beide Statoren 4 in der Schliessstellung sind, ist das Rotorgehäuse 2 sowohl gegen Drehbewegungen als auch gegen translatorische Bewegungen gesichert.

Es versteht sich, dass die beiden Statoren 4 an einer Stützstruktur 400 (Fig. 3), beispielsweise an einer Gehäusewand, fixiert sind.

Anhand der Fig. 11 - Fig. 22 werden nun verschieden Varianten für die Ausgestaltung des Stators 4 beschrieben, die für die vorangehend beschriebenen Ausführungsbeispiele geeignet sind. Dabei werden sowohl Varianten des Stators 4 für die Ausgestaltung des elektromagnetischen Drehantriebs 1 als Tempelmotor beschrieben als auch Varianten für die Ausgestaltung des elektromagnetischen Drehantriebs 1 als Flachmotor oder Radialmotor. Bei allen Varianten ist der Stator 4 vorzugsweise als Lager- und Antriebsstator ausgestaltet, mit welchem der Rotor 3 im Betriebszustand berührungslos magnetisch zur Rotation antreibbar und berührungslos magnetisch bezüglich des Stators 4 lagerbar ist. Vorzugsweise ist der elektromagnetische Drehantrieb 1 nach dem weiter vorne beschriebenen Prinzip des lagerlosen Motors ausgestaltet und wird nach diesem Prinzip betrieben. Bei Ausführungsbeispielen des erfindungsgemässen elektromagnetischen Drehantriebs 1, bei denen mehr als ein Stator 4 vorgesehen ist, ist mindestens einer der Statoren 4 als Lager- und Antriebsstator für den Betrieb als lagerloser Motor ausgestaltet.

In allen Fig. 11 - Fig. 22 ist jeweils das Statorgehäuse 5 aus Gründen einer besseren Übersicht nicht dargestellt. Es versteht sich, dass bei allen Varianten der erste Statorteil 48 ein erstes Gehäuseteil 58 (Fig. 5, Fig. 6) umfasst, und der zweite Statorteil 49 ein zweites Gehäuseteil 59. Vorzugsweise sind das erste Gehäuseteil 58 und das zweite Gehäuseteil 59 jeweils als Kapselung des ersten Statorteils 48 bzw. des zweiten Statorteils 49 ausgestaltet. Insbesondere können der erste Statorteil 48 und der zweite Statorteil 49 jeweils hermetisch gekapselt ausgestaltet sein.

Für alle beschriebenen Varianten für die Ausgestaltung des Stators 4 kann das jeweilige Statorgehäuse 5 in gleicher oder in sinngemäss gleicher Weise ausgestaltet sein, wie dies anhand der Fig. 5 und Fig. 6 erläutert ist.

Es versteht sich, dass die für eine spezifische Variante beschriebenen Massnahmen oder Ausgestaltungen in gleicher oder in sinngemäss gleicher Weise auch mit den anderen Varianten kombiniert werden können, auch wenn sie dort nicht explizit erwähnt sind.

Fig. 11 zeigt eine perspektivische Darstellung einer ersten Variante für die Ausgestaltung des Stators 4, wobei der Stator in der Schliessstellung ist. Fig. 12 zeigt diesen Stator 4 in der Offenstellung.

Die erste Variante des Stators 4 ist geeignet für eine Ausgestaltung des elektromagnetischen Drehantriebs 1 als Tempelmotor.

Der Stator 4 umfasst eine Mehrzahl, hier fünf, von den Spulenkernen 45, von denen jeder den stabförmigen Längsschenkel 46 umfasst, welcher sich von dem ersten Ende 461 in axialer Richtung A bis zu dem zweiten Ende 462 erstreckt, sowie den Querschenkel 47, welcher an dem zweiten Ende 462 des Längsschenkels 46 angeordnet ist. Jeder Querschenkel 47 erstreckt sich in radialer Richtung auf den magnetisch wirksamen Kern 31 des Rotors 3 zu und wird durch eine dem magnetisch wirksamen Kern 31 zugewandte Stirnfläche begrenzt.

Die fünf Spulenkerne 45 sind in der Schliessstellung des Stators 4 äquidistant auf einer Kreislinie angeordnet, sodass die Stirnflächen der Querschenkel 47 den magnetisch wirksamen Kern 31 des Rotors 3 umgeben. Der magnetisch wirksame Kern 31 des Rotors 3 ist als permanentmagnetische Scheibe oder als permanentmagnetischer Ring ausgestaltet, der vorzugsweise diametral magnetisiert ist.

Jeder Querschenkel 47 bildet jeweils einen Statorpol 41. Die Statorpole 41 sind um den magnetisch wirksamen Kern 31 des Rotors3 herum angeordnet, das heisst, im Betriebszustand ist der magnetisch wirksame Kern 31 des Rotors 3 von den Statorpolen 41 umgeben.

Die ersten Enden 461 der Längsschenkel 46 sind in Umfangsrichtung gesehen durch den ringförmigen Rückschluss 42 verbunden. Der Rückschluss 42 ist vorzugsweise ringförmig ausgestaltet oder umfasst mehrere Segmente, welche die Längsschenkel 46 miteinander verbinden. Sowohl der Rückschluss 42 als auch die Spulenkerne 45 des Stators 4 sind jeweils aus einem weichmagnetischen Material gefertigt, weil sie als Flussleitelemente zur Führung des magnetischen Flusses dienen. Geeignete weichmagnetische Materialien für die Spulenkerne 45 und den Rückschluss 42 sind beispielsweise ferromagnetische oder ferrimagnetische Materialien, also insbesondere Eisen, Nickel-Eisen, Kobalt-Eisen Silizium-Eisen oder Mu-Metall. Hierbei ist für den Stator 4 eine Ausgestaltung als Statorblechpaket bevorzugt, bei welcher die Spulenkerne 45 und der Rückschluss 42 geblecht ausgestaltet sind, das heisst sie bestehen aus mehreren dünnen Blechelementen, die gestapelt sind. Der Rückschluss 42 kann beispielsweise auch mehrere Segmente umfassen, die jeweils zwischen zwei benachbarten Spulenkernen 45 angeordnet sind.

Der magnetisch wirksame Kern 31 rotiert im Betriebszustand um die Solldrehachse, welche die axiale Richtung A definiert, wobei der Rotor 3 berührungslos magnetisch angetrieben und berührungslos magnetisch bezüglich des Stators 4 gelagert ist. Dabei wird die radiale Position des Rotors 3 bzw. des magnetisch wirksamen Kerns 31 so geregelt, dass er sich in einer zentrierten Position zwischen den Statorpolen 41 befindet. Für die Regelung des Antriebs und der Lage des Rotors 3 sind eine Mehrzahl von Sensoren 44 vorgesehen, beispielsweise Positionssensoren, die vorzugsweise bezüglich der Umfangsrichtung jeweils zwischen den Stirnflächen zweier benachbarter Querschenkel 47 angeordnet sind. Alle Sensoren 44, hier also fünf Sensoren 44, sind mit der Kontrolleinrichtung 80 signalverbunden, die vorzugsweise auch innerhalb des Statorgehäuses 5, beispielsweise zwischen dem Rückschluss 42 und einem Boden des Statorgehäuses 5 angeordnet ist, oder auch radial innenliegend bezüglich des Rückschlusses 42.

Um die für den magnetischen Antrieb und die magnetische Lagerung des magnetisch wirksamen Kerns 31 des Rotors 3 notwendigen elektromagnetischen Drehfelder zu erzeugen, tragen die Längsschenkel 46 die Wicklungen 6, die jeweils als konzentrierte Wicklung 6 ausgestaltet sind. Um jeden Längsschenkel 46 herum ist jeweils genau eine konzentrierte Wicklung 6 angeordnet. Mit diesen konzentrierten Wicklungen 6 werden im Betriebszustand diejenigen elektromagnetischen Drehfelder erzeugt, mit welchen ein Drehmoment auf den Rotor 3 bewirkt wird, und mit welchen eine beliebig einstellbare Querkraft in radialer Richtung auf den magnetisch wirksamen Kern 31 des Rotors 3 ausübbar ist, sodass die radiale Position des magnetisch wirksamen Kerns 31, also seine Position in der zur axialen Richtung A senkrechten radialen Ebene, aktiv steuerbar bzw. regelbar ist.

Bei der hier beschriebenen ersten Variante wird also die Antriebs- und Lagerfunktion mit nur einem einzigen Wicklungssystem, nämlich mit den fünf konzentrierten Wicklungen 6 durchgeführt. Dazu wird in der Kontrolleinrichtung 80 der jeweilige Wert für den Antriebs- und den Steuerstrom ermittelt. Die beiden Werte werden dann rechnerisch -also z. B. mit Hilfe von Software - addiert bzw. überlagert und der sich daraus ergebende Gesamtstrom in die jeweilige konzentrierte Wicklung 6 eingeprägt.

Dazu ist die Kontrolleinheit 80 mit jeder Wicklung 6 signalverbunden und umfasst die Leistungselektronik zur Versorgung und zur Ansteuerung der Wicklungen 6.

Der Stator 4 umfasst den ersten Statorteil 48, der hier drei Spulenkerne 45 mit den darauf angeordneten Wicklungen 6, einen Teil des Rückschlusses 42 und vier Positionssensoren 44 umfasst. Diese Komponenten des ersten Statorteils 48 sind in dem ersten Gehäuseteil 58 (siehe z. B. Fig. 6) angeordnet und durch dieses erste Gehäuseteil 58 vorzugsweise gekapselt. Ferner können die Kontrolleinrichtung 80 oder Teile der Kontrolleinrichtung 80 in dem ersten Gehäuseteil 58 angeordnet sein.

Der Stator 4 umfasst ferner den zweiten Statorteil 49, der hier zwei Spulenkerne 45 mit den darauf angeordneten Wicklungen 6, einen Teil des Rückschlusses 42 und einen der Positionssensoren 44 umfasst. Diese Komponenten des zweiten Statorteils 49 sind in dem zweiten Gehäuseteil 59 (siehe z. B. Fig. 6) angeordnet und durch dieses zweite Gehäuseteil 59 vorzugsweise gekapselt. Ferner können die Kontrolleinrichtung 80 oder Teile der Kontrolleinrichtung 80 in dem zweiten Gehäuseteil 59 angeordnet sein.

Die beiden Gehäuseteile 58, 59 sind über das Gelenk 55 miteinander verbunden und beispielsweise so ausgestaltet, wie dies anhand der Fig. 5 und Fig. 6 erläutert wurde. Somit ist der Stator 4 öffenbar mit dem ersten Statorteil 48 und dem zweiten Statorteil 49 ausgestaltet, welche relativ zueinander bewegbar sind, sodass der Stator 4 aus der Offenstellung in die Schliessstellung zugeklappt und aus der Schliessstellung in die Offenstellung aufgeklappt werden kann.

Fig. 13 zeigt in einer perspektivischen Darstellung einer Variante für die Ausführung des Statorgehäuses 5 in der Offenstellung. Diese Ausgestaltung des Statorgehäuses 5 des Stators 4 ist insbesondere für solche Ausführungsformen des Stators 4 geeignet, bei welche der Stator 4 eine gerade Anzahl von Statorpolen 41 oder Spulenkernen 45 aufweist. Bei der in Fig. 13 dargestellten Variante sind das erste Gehäuseteil 58 und das zweite Gehäuseteil 59 in Umfangsrichtung gesehen im Wesentlichen gleich lang ausgestaltet, sodass beide Gehäuseteile 58, 59 die gleiche Anzahl von Statorpolen 41 bzw. von Spulenkernen 45 aufnehmen und vorzugsweise einkapseln können. Die beiden Gehäuseteile 58 und 59 sind jeweils in Form eines Halbzylinders ausgestaltet. Ansonsten ist das in Fig. 13 dargestellte Statorgehäuse 5 in sinngemäss gleicher Weise ausgestaltet wie dies anhand der Fig. 5 und Fig. 6 erläutert ist.

Fig. 14 zeigt in einer perspektivischen Darstellung einer zweiten Variante für die Ausgestaltung des Stators 4, wobei der Stator 4 in der Schliessstellung ist. Diese zweite Variante ist ebenfalls für eine Ausgestaltung des elektromagnetischen Drehantriebs 1 als Tempelmotor geeignet. Im Unterschied zu der ersten Variante (Fig. 11, Fig. 12) hat die zweite Variante eine gerade Anzahl von Spulenkernen 45, nämlich sechs Spulenkerne 45. Ausserdem sind auf jedem Spulenkern 45 jeweils zwei Spulen vorgesehen, nämlich jeweils eine Antriebsspule 61 und eine Steuerspule 62. Die Antriebsspulen 61 dienen zur Erzeugung des Antriebsfelds, und die Steuerspulen dienen zur Erzeugung des Steuerfelds. Bei der zweiten Variante sind also zwei unterschiedliche Wicklungssysteme zur Erzeugung des Antriebsfelds und zur Erzeugung des Steuerfelds vorgesehen.

Bei einer geraden Anzahl von Statorpolen 41 bzw. Spulenkernen 45 ist der Stator 4 vorzugsweise symmetrisch geteilt, das heisst der erste Statorteil 48 und der zweite Statorteil 49 umfassen die gleiche Anzahl von Statorpolen 41 bzw. Spulenkernen 45.

Bei der zweiten Variante umfasst der Stator 4 den ersten Statorteil 48, der drei Spulenkerne 45 mit jeweils einer darauf angeordneten Antriebsspule 61 und einer darauf angeordneten Steuerspule 62, einen Teil, nämlich im Wesentlichen sie Hälfte des Rückschlusses 42 und vier Positionssensoren 44 umfasst. Diese Komponenten des ersten Statorteils 48 sind in dem ersten Gehäuseteil 58 (siehe z. B. Fig. 13) angeordnet und durch dieses erste Gehäuseteil 58 vorzugsweise gekapselt. Ferner können die Kontrolleinrichtung 80 (in Fig. 14 nicht dargestellt) oder Teile der Kontrolleinrichtung 80 in dem ersten Gehäuseteil 58 angeordnet sein.

Der Stator 4 umfasst ferner den zweiten Statorteil 49, der drei Spulenkerne 45 mit jeweils einer darauf angeordneten Antriebsspule 61 und einer darauf angeordneten Steuerspule 62, einen Teil, nämlich im Wesentlichen sie Hälfte des Rückschlusses 42 und zwei Positionssensoren 44 umfasst. Diese Komponenten des zweiten Statorteils 49 sind in dem zweiten Gehäuseteil 59 (siehe z. B. Fig. 13) angeordnet und durch dieses zweite Gehäuseteil 59 vorzugsweise gekapselt. Ferner können die Kontrolleinrichtung 80 oder Teile der Kontrolleinrichtung 80 in dem zweiten Gehäuseteil 58 angeordnet sein.

Aufgrund dergleichen Anzahl von Statorpolen 41 bzw. Spulenkernen 45 im ersten Statorteil 48 und im zweiten Statorteil 49 ist das Statorgehäuse 5 vorzugsweise wie in Fig. 13 dargestellt ausgestaltet.

Es versteht sich, dass bei der Ausgestaltung mit separaten Antriebsspulen 61 und separaten Steuerspulen 62 auch Ausführungsformen mit einer ungeraden Anzahl von Statorpolen 41 oder Spulenkernen 45 möglich sind. Bei den Ausgestaltungen mit nur einer Wicklung 6 auf jedem Spulenkern 45 bzw. auf jedem Statorpol 41 ist es natürlich auch möglich, eine gerade Anzahl von Statorpolen 41 oder Spulenkernen 45 vorzusehen (siehe z. B. Fig. 2). Ferner sind Ausgestaltungen mit einer geraden oder mit einer ungeraden Anzahl von Statorpolen 41 oder Spulenkernen 45 möglich, wobei die Anzahl grösser als sechs ist.

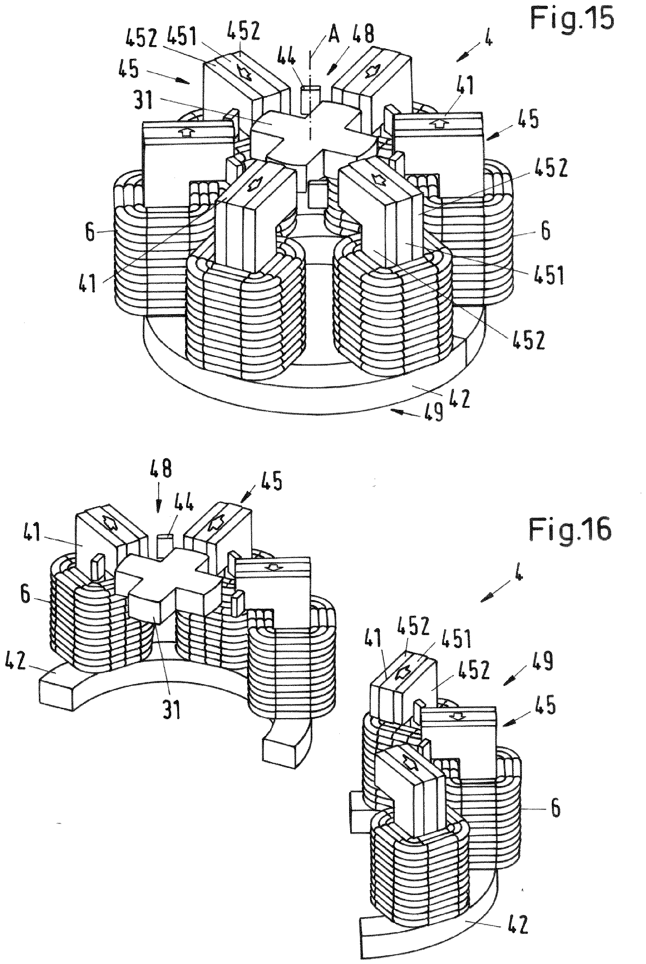

Fig. 15 zeigt eine perspektivische Darstellung einer dritten Variante für die Ausgestaltung des Stators 4 und des Rotors 3, wobei der Stator 4 in der Schliessstellung ist. Fig. 16 zeigt diese dritte Variante in der Offenstellung. Diese dritte Variante ist ebenfalls für eine Ausgestaltung des elektromagnetischen Drehantriebs 1 als Tempelmotor geeignet. Bei der dritten Variante sind wiederum - mit beispielhaftem Charakter- sechs Spulenkerne 45 vorgesehen. Um den Längsschenkel 46 jedes Spulenkerns 45 ist genau eine Wicklung 6 angeordnet.

Bei der dritten Variante ist der Stator 4 permanentmagnetisch ausgestaltet. Eine solche permanentmagnetische Ausgestaltung des Stators 4 ist beispielsweise in der EP 3 232 549 A1 der gleichen Anmelderin offenbart.

Jeder Spulenkern 45 umfasst jeweils einen permanentmagnetischen Teil 451, der zwischen zwei permanentmagnetfreien Teilen 452 eingeschlossen ist, wobei beide permanentmagnetfreien Teile 452 und der permanentmagnetische Teil 451 jeweils L-förmig ausgestaltet sind.

Sowohl jeder der permanentmagnetfreien Teile 452 als auch jeder der permanentmagnetischen Teile 451 haben also eine L-förmige Ausgestaltung, wobei die beiden Grenzflächen der beiden permanentmagnetfreien Teile 452, die an den permanentmagnetischen Teil 451 angrenzen, jeweils deckungsgleich mit den sie kontaktierenden Grenzflächen des permanentmagnetischen Teils 451 ausgestaltet sind. Somit sind die beiden permanentmagnetfreien Teile 452 jedes Spulenkerns 45 durch den jeweils zwischen ihnen liegenden permanentmagnetischen Teil 451 vollkommen voneinander getrennt.

Das bedeutet, dass sich die permanentmagnetischen Teile 451 und die beiden permanentmagnetfreien Teile 452 jedes Spulenkerns 45 jeweils auch durch den Querschenkel 47 erstrecken, und auch im Querschenkel 47 der permanentmagnetische Teil 451 zwischen den beiden permanentmagnetfreien Teilen 452 angeordnet ist.

Wie dies in den Fig. 15 und Fig. 16 die Pfeile ohne Bezugszeichen in den permanentmagnetischen Teilen 451 zeigen, sind die permanentmagnetischen Teile 451 jeweils in Umfangsrichtung des Stators 4 polarisiert bzw. magnetisiert, d.h. jeder permanentmagnetische Teil 451 weist eine Magnetisierung auf, die senkrecht zur radialen Richtung und senkrecht zur axialen Richtung A ausgerichtet ist. Die permanentmagnetischen Teile 451 benachbarter Spulenkerne 4 sind dabei jeweils in umgekehrter Richtung magnetisiert, das heisst jeder permanentmagnetische Teil 451 eines Spulenkerns 45 ist in Umfangsrichtung gesehen von zwei permanentmagnetischen Teilen 451 benachbarter Spulenkerne 45 umgeben, die jeweils in entgegengesetzter Richtung magnetisiert sind wie er selbst.

Durch die Ausgestaltung des Stators 4 des Tempelmotors, die Permanentmagnete im Stator 4 umfasst, ist es möglich, den gesamten magnetischen Fluss im Stator 4 zu generieren. Hierdurch wird es insbesondere möglich, dass der Rotor 3 nicht zur Generierung des magnetischen Flusses beitragen, sondern diesen nur leiten bzw. führen muss. Somit kann im Rotor 3 auf Permanentmagnete bzw. sehr hartmagnetische Stoffe zur Flussgenerierung verzichtet werden. Da der Rotor 3 Bestandteil der Einmalvorrichtung 100 ist, ist es aus wirtschaftlichen Gründen und unter Umweltaspekten vorteilhaft, dass der Rotor 3 frei von Permanentmagneten ausgestaltet werden kann.

Auch ist der Rotor 3 spulenfrei ausgestaltet, d. h. auf dem Rotor 3 sind keine Wicklungen vorgesehen.

Der magnetisch wirksame Kern 31 des Rotors kann also spulenfrei und ohne Permanentmagnete, beispielsweise als Reluktanzläufer ausgestaltet werden. Vorzugsweise ist der magnetisch wirksame Kern 31 des Rotors 3 aus einem weichmagnetischen Material gefertigt, beispielsweise aus Eisen, Nickel-Eisen, Kobalt-Eisen, Silizium-Eisen oder Mu-Metall. Bei der Ausgestaltung als Reluktanzläufer ist der magnetisch wirksamen Kern 31 beispielsweise - wie in Fig. 15 und Fig. 16 gezeigt - als scheibenförmiges Kreuz mit vier ausgeprägten Rotorzähnen ausgestaltet.

Bezüglich weiterer Erläuterungen zur Ausgestaltung des Stators 4 mit Permanentmagneten wird auf die EP 3 232 549 verwiesen.

Fig. 17 zeigt eine perspektivische Darstellung einer vierten Variante für die Ausgestaltung des Stators 4, wobei der Stator 4 in der Schliessstellung ist. Fig. 18 zeigt diese vierte Variante mit dem Stator 4 in der Offenstellung. Diese vierte Variante ist für eine Ausgestaltung des elektromagnetischen Drehantriebs 1 als Flachmotor bzw. als Radialmotor geeignet.

Aus Gründen der besseren Übersicht und weil es für das Verständnis ausreicht, ist in den Fig. 17 und Fig. 18 der magnetisch wirksame Kern 31 nicht dargestellt. Dieser kann beispielsweise in sinngemäss gleicher Weise ausgestaltet sein, wie dies bezüglich Fig. 4 beschrieben ist. Das heisst, der magnetisch wirksame Kern 31 ist dann als permanentmagnetische Scheibe oder als permanentmagnetischer Ring ausgestaltet und diametral magnetisiert.

In den Fig. 17 und Fig. 18 ist der Stator 4 für eine Ausgestaltung als Flachmotor gezeigt, wobei der Stator 4 insgesamt sechs ausgeprägte Statorpole 41 aufweist, die sich jeweils von dem radial aussenliegenden ringförmigen Rückschluss 42 in radialer Richtung nach innen auf den magnetischen Kern 31 (nicht dargestellt) des Rotors 3 hin erstrecken. Jeder Statorpol 41 ist in der radialen Ebene angeordnet und wird jeweils durch eine dem magnetisch wirksamen Kern 31 des Rotors 3 zugewandte Stirnfläche begrenzt. Während des Betriebs des elektromagnetischen Drehantriebs 1 ist es die Soll-Position, dass der magnetisch wirksame Kern 31 zwischen den Stirnflächen der Statorpole 41 zentriert ist.

Um die für den magnetischen Antrieb und die magnetische Lagerung des Rotors 3 notwendigen elektromagnetischen Drehfelder zu erzeugen, tragen die Statorpole 41 die Wicklungen 6, die auch hier wieder als konzentrierte Wicklungen 6 beispielsweise so ausgestaltet sind, dass um jeden Statorpol 41 herum jeweils genau eine konzentrierte Wicklung 6 gewickelt ist, sodass jede konzentrierte Wicklung 6 in der radialen Ebene angeordnet ist. Mit diesen konzentrierten Wicklungen 6 werden im Betriebszustand die elektromagnetischen Drehfelder für den Antrieb und die Lagerung des Rotors 3 erzeugt.

Bei der hier dargestellten Ausführungsform sind die Wicklungen 6 als kombinierte Wicklungen dargestellt, das heisst, es sind keine separaten Antriebsspulen und Steuerspulen vorgesehen. Natürlich sind auch solche Ausgestaltungen des Flachmotors möglich, bei denen separate Antriebsspulen und separate Steuerspulen vorgesehen sind, beispielsweise in sinngemäss gleicher Weise, wie dies anhand von Fig. 14 beschrieben ist. Bei der Ausgestaltung mit separaten Antriebs- und Steuerspulen ist es bevorzugt, dass auf jedem Statorpol 41 jeweils genau eine Steuerspule und genau eine Antriebsspule vorgesehen ist.

Es sei darauf hingewiesen, dass die Ausgestaltung mit sechs Statorpolen 41 beispielhaft zu verstehen ist. Auch bei der Ausgestaltung als Flachmotor können beispielsweise nur fünf oder auch mehr als sechs Statorpole 41 vorgesehen sein.

Auch bei der Ausgestaltung als Flachmotor umfasst der Stator 4 eine Mehrzahl von Sensoren 44, beispielsweise Positionssensoren, die vorzugsweise bezüglich der Umfangsrichtung jeweils zwischen zwei benachbarten Statorpolen 41 angeordnet sind. Insgesamt sind sechs Sensoren 44 vorgesehen, von denen jeder jeweils zwischen zwei in Umfangsrichtung benachbarten Statorpolen 41 vorgesehen ist. Alle Sensoren 44 sind mit der Kontrolleinrichtung 80 signalverbunden, welche die Leistungselektronik für die Ansteuerung und die Versorgung der Wicklungen 6 sowie die Signalverarbeitungselektronik umfasst.

Die Kontrolleinrichtung 80 umfasst einen oder mehrere Platinen bzw. PCBs (PCB: printed circuit board) 801, die auf dem Rückschluss 42 bzw. einer nicht dargestellten Ummantelung des Rückschlusses 42 angeordnet sind. Jeder Sensor 44 ist mit einem PCB 801 verbunden, sodass die Signalauswertung und die Ansteuerung der Sensoren 44 in den PCBs 801 erfolgen kann. Die PCBs können insbesondere auch so ausgestaltet sein, dass die Sensoren 44 jeweils direkt auf einem PCB 801 angeordnet sind. Hierzu ist es möglich, wie in Fig. 17 und Fig. 18 gezeigt, den jeweiligen PCB 801 mit gebogenen Teilen 802 auszugestalten, beispielsweise als flexiblen, das heisst biegbaren PCB 801. Die PCBs 801 können auch weitere Komponenten umfassen, beispielsweise Leitungen für die Versorgung und die Ansteuerung der Wicklungen 6.

Bei einer geraden Anzahl von Statorpolen 41 ist der Stator 4 vorzugsweise symmetrisch geteilt, das heisst der erste Statorteil 48 und der zweite Statorteil 49 umfassen die gleiche Anzahl von Statorpolen 41. Das Statorgehäuse 5 ist dann vorzugsweise sinngemäss so ausgestaltet wie es unter Bezugnahme auf Fig. 13 erläutert ist.

Bei der vierten Variante umfasst der Stator 4 den ersten Statorteil 48, der drei Statorpole 41 mit jeweils einer darauf angeordneten Wicklung 6, einen Teil, nämlich im Wesentlichen die Hälfte des Rückschlusses 42, vier Positionssensoren 44 sowie einen PCB 801 für diese Positionssensoren 44 umfasst. Diese Komponenten des ersten Statorteils 48 sind in dem ersten Gehäuseteil 58 (siehe z. B. Fig. 13) angeordnet und durch dieses erste Gehäuseteil 58 vorzugsweise gekapselt. Ferner können weitere Teile der Kontrolleinrichtung 80 in dem ersten Gehäuseteil 58 angeordnet sein.

Der Stator 4 umfasst ferner den zweiten Statorteil 49, der drei Statorpole 41 mit jeweils einer darauf angeordneten Wicklung 6, einen Teil, nämlich im Wesentlichen die Hälfte des Rückschlusses 42, zwei Positionssensoren 44 sowie einen PCB 801 für diese Positionssensoren 44 umfasst. Diese Komponenten des zweiten Statorteils 49 sind in dem zweiten Gehäuseteil 59 (siehe z. B. Fig. 13) angeordnet und durch dieses zweite Gehäuseteil 59 vorzugsweise gekapselt. Ferner können weitere Teile der Kontrolleinrichtung 80 in dem zweiten Gehäuseteil 58 angeordnet sein.

Wie dies insbesondere in Fig. 18 zu erkennen ist, sind in den Grenzflächen des Rückschlusses 42, also dort wo die Teilung ist zwischen dem Teil des Rückschlusses 42, der zum ersten Statorteil 48 gehört, und dem Teil des Rückschlusses 42, der zum zweiten Statorteil 49 gehört, jeweils eine Ausnehmung 571 und eine Erhebung 572 als mechanische Zentrierung vorgesehen. In sinngemäss gleicher Weise wie dies anhand von Fig. 5 und Fig. 6 erläutert ist, sind die Ausnehmungen 571 und die Erhebungen 572 so ausgestaltet und angeordnet, dass jede Erhebung 572 in der Schliessstellung des Stators 4 jeweils in eine der Ausnehmungen 571 eingreift, vorzugsweise passgenau eingreift, und damit die beiden Statorteile 48, 49 relativ zueinander zentriert. Die im Rückschluss 42 angeordneten Ausnehmungen 571 und Erhebungen 572 können alternativ oder ergänzend zu den am Statorgehäuse 5 angeordneten Ausnehmungen 571 und Erhebungen 572 (siehe Fig. 6) vorgesehen sein.

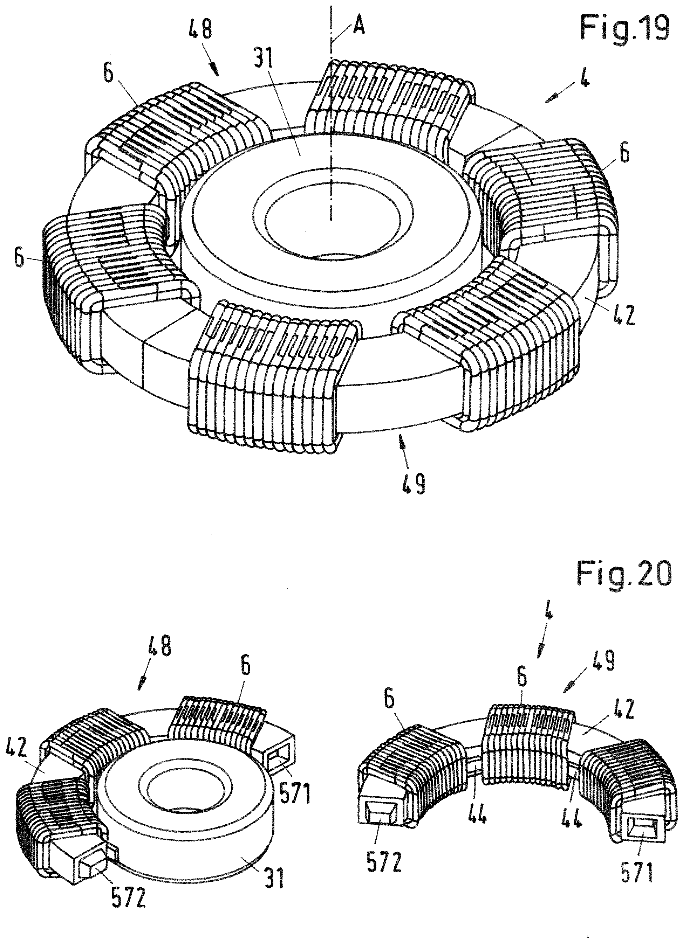

Fig. 19 zeigt eine perspektivische Darstellung einer fünften Variante für die Ausgestaltung des Stators 4 und des Rotors 3, wobei der Stator 4 in der Schliessstellung ist. Fig. 20 zeigt diese Variante mit dem Stator 4 in der Offenstellung. Da es für das Verständnis ausreichend ist, ist von dem Rotor 3 nur der magnetisch wirksame Kern 31 dargestellt. Ferner sind die Sensoren 44 und die Kontrolleinrichtung 80 nicht dargestellt.

Die fünfte Variante ist für die Ausgestaltung des elektromagnetischen Drehantriebs 1 als Flachmotor geeignet. Der magnetisch wirksame Kern 31 ist wiederum als permanentmagnetische Scheibe oder als permanentmagnetischer Ring ausgestaltet und vorzugsweise diametral magnetisiert.

Ein wesentlicher Unterschied zu der vierten Variante ist es, dass die fünfte Variante für einen nutenlosen elektromagnetischen Drehantrieb 1 ausgestaltet ist. Es sind keine ausgeprägten Statorpole 41 oder Spulenkerne 45 vorgesehen, sondern die Wicklungen 6 sind jeweils als Toroidspulen ausgestaltet, die um den Rückschluss 42 herum gewickelt sind. In der Schliessstellung des Stators (Fig. 20) ist der Stator 4 im Wesentlichen ringförmig.

Es sind insgesamt sechs Wicklungen 6 vorgesehen, die äquidistant bezüglich der Umfangsrichtung auf dem ringförmigen Rückschluss 42 angeordnet sind und den radial innenliegenden magnetische Kern 31 umgeben. Es sind natürlich auch Ausgestaltungen mit weniger als sechs, beispielsweise fünf, oder mit mehr als sechs Wicklungen 6 möglich.

Bei der hier dargestellten Ausführungsform sind die Wicklungen 6 als kombinierte Wicklungen dargestellt, das heisst, es sind keine separaten Antriebsspulen und Steuerspulen vorgesehen. Natürlich sind auch solche Ausgestaltungen des nutenlosen Motors möglich, bei denen separate Antriebsspulen und separate Steuerspulen vorgesehen sind. Diese sind dann beispielsweise jeweils übereinander gewickelt.

Bei der Ausgestaltung mit sechs Wicklungen 6 bzw. allgemeiner mit einer geraden Anzahl von Wicklungen 6, ist der Stator 4 vorzugsweise symmetrisch geteilt, sodass der erste Statorteil 48 die gleiche Anzahl an Wicklungen 6 umfasst wie der zweite Statorteil 49.

Die Ausgestaltung des elektromagnetischen Drehantriebs 1 als nutenloser Motor eignet sich insbesondere für Anwendungen, bei der hohe Drehzahlen des Rotors 3 erforderlich sind, oder wo es auf möglichst geringe Verluste ankommt.

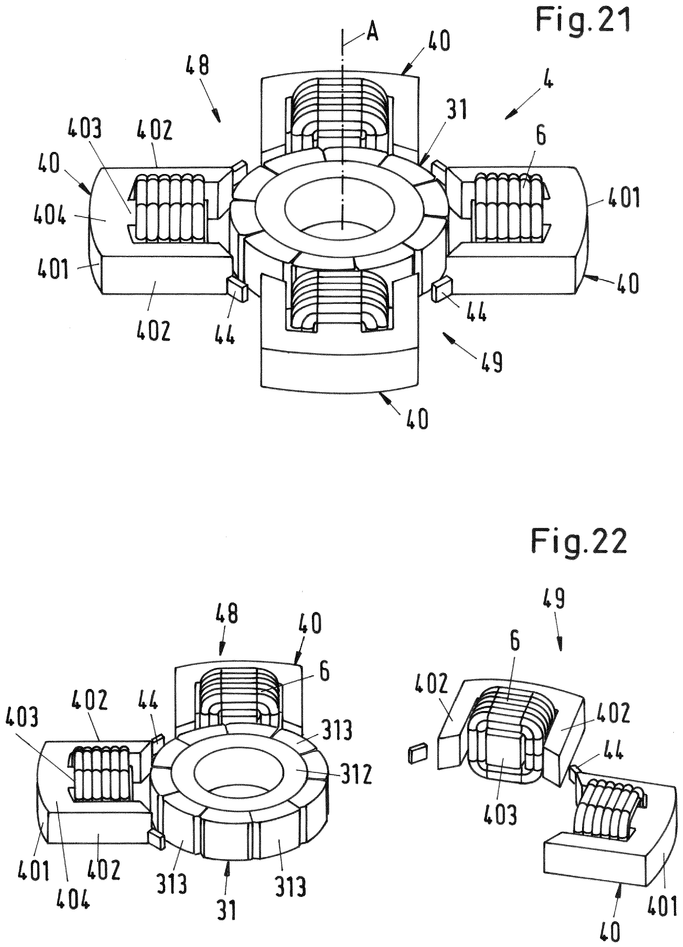

Fig. 21 eine perspektivische Darstellung einer sechsten Variante für die Ausgestaltung des Stators 4 und des Rotors 3, wobei der Stator 4 in der Schliessstellung ist. Fig. 22 zeigt die sechste Variante mit dem Stator 4 in der Offenstellung.

Die sechste Variante ist für die Ausgestaltung des elektromagnetischen Drehantriebs 1 als Flachmotor geeignet. Im Speziellen ist die sechste Variante für eine Ausgestaltung des elektrischen Drehantriebs 1 als Segmentmotor geeignet.

Der Stator 4 umfasst eine Mehrzahl - hier mit beispielhaftem Charakter vier - Statorsegmente 40, die äquidistant bezüglich der Umfangsrichtung um den radial innenliegenden magnetische Kern 31 des Rotors 3 herum angeordnet sind. Die einzelnen Statorsegmente 40 sind nicht direkt magnetisch miteinander verbunden. Es gibt also keine direkte magnetische Verbindung zwischen den Statorsegmente 40, so wie sie beispielsweise in den vorangehend beschriebenen Varianten durch den Rückschluss 42 realisiert ist.

Jedes Statorsegment 40 umfasst einen Segmentkern 401, der im Wesentlichen E-förmig ausgestaltet ist, mit zwei aussenliegenden Schenkeln 402 und einem zentralen Schenkel 403, der bezüglich der Umfangsrichtung zwischen den beiden aussenliegenden Schenkeln 402 angeordnet ist. Ferner ist ein ringsegmentförmige Basis 404 vorgesehen, welche die radial aussenliegenden Enden der beiden aussenliegenden Schenkel 402 und des zentralen Schenkels 403 miteinander verbindet. Die beiden aussenliegenden Schenkel 402 und der zentrale Schenkel 403 erstrecken sich also jeweils von der Basis 404 in radialer Richtung auf den radial innenliegend angeordneten magnetisch wirksamen Kern 31 des Rotors 3 zu. In sinngemäss gleicher Weise wir dies vorangehend für den Rückschluss 42 erläutert wurde, ist jeder Segmentkern 401 jeweils aus einem weichmagnetischen Material gefertigt, wobei eine Ausgestaltung als Blechpaket bevorzugt ist.

Auf dem zentralen Schenkel 403 ist jeweils genau eine Wicklung 6 angeordnet, sodass der Stator 4 insgesamt vier Wicklungen 6 aufweist. Die Wicklungen 6 sind als kombinierte Wicklungen ausgestaltet, das heisst, es sind keine separaten Antriebsspulen und Steuerspulen vorgesehen.

Wie bereits erwähnt, sind die Statorsegmente 40 magnetisch nicht direkt miteinander verbunden. In jedem Statorsegment 40 wird der von der Wicklung generierte magnetische Fluss über den zentralen Schenkel 403 in den magnetisch wirksamen Kern 31 geführt und über die beiden aussenliegenden Schenkel 402 wieder in das Statorsegment 40 zurückgeführt oder umgekehrt.

Der ringförmig ausgestaltete magnetisch wirksame Kern 31 umfasst einen radial innenliegenden, ringförmigen Rotorrückschluss 312 aus einem weichmagnetischen Material und eine Mehrzahl von Permanentmagneten 313, hier zehn Permanentmagnete 313, die radial aussenliegend entlang des Umfangs des Rotorrückschlusses 312 auf dem Rotorrückschluss 313 angeordnet sind.

Bei der Ausgestaltung mit vier Statorsegmenten 40, bzw. allgemeiner mit einer geraden Anzahl von Statorsegmenten 40, ist der Stator 4 vorzugsweise so ausgestaltet, sodass der erste Statorteil 48 die gleiche Anzahl an Statorsegmenten 40 umfasst wie der zweite Statorteil 49.

Anhand der Fig. 23-25 werden nun verschiedene Varianten für die Ausgestaltung der Kontrolleinrichtung 80 erläutert. Die Fig. 23 bis Fig. 25 zeigen jeweils schematische Darstellungen.

Die Kontrolleinrichtung 80 umfasst die für den Betrieb des elektromagnetischen Drehantriebs 1 notwendigen Kontroll-, Regel- und Ansteuereinrichtungen, also insbesondere die Leistungselektronik für die Ansteuerung und die Versorgung der Wicklungen 6, 61, 62. Mit der Leistungselektronik werden in den Wicklungen 6, 61, 62 die Ströme für die Erzeugung der elektromagnetischen Drehfelder generiert. Ferner umfasst die Kontrolleinrichtung 80 die Signalverarbeitungselektronik, mit welcher die Sensoren 44 angesteuert und die von den Sensoren 44 übermittelten Signale ausgewertet werden. Auch führt die Kontrolleinrichtung 80 Regelfunktionen durch, beispielsweise die aktive magnetische Regelung der radialen Position des magnetisch wirksamen Kerns 31 des Rotors 3 in der radialen Ebene. Wie bereits erwähnt, können zumindest Teile der Kontrolleinrichtung als PCB ausgestaltet sein, beispielsweise als die PCBs 801, die im Zusammenhang mit den Fig. 17 und Fig. 18 erläutert sind.

Fig. 23 zeigt eine Variante, bei welcher die gesamte Kontrolleinrichtung 80 ausserhalb des Statorgehäuses 5 angeordnet ist und über eine elektrische Leitung L mit dem Statorgehäuse 5 bzw. mit den darin angeordneten Komponenten des Stators 4 verbunden ist.

Fig. 24 zeigt eine Variante, bei welcher die Kontrolleinrichtung 80 in einem der beiden Gehäuseteile 58, 59, hier in dem ersten Gehäuseteil 58, angeordnet ist. Die Verbindung der Kontrolleinheit 80 mit dem im zweiten Gehäuseteil 59 angeordneten zweiten Statorteil 49 erfolgt dann beispielsweise über die elektrische Verbindung S, welche durch das Gelenk 55 hindurchgeführt ist.

Fig. 25 zeigt eine Variante, bei welcher die Kontrolleinrichtung 80 auf die beiden Gehäuseteile 58, 59 verteilt ist. Hierbei ist es beispielsweise möglich, dass die gesamte Leistungselektronik der Kontrolleinrichtung 80 in einem der beiden Gehäuseteile 58, 59 angeordnet ist, und die Elektronik für die Signalverarbeitung auf die beiden Gehäuseteile 58, 59 verteilt ist. Es ist aber auch möglich, dass sowohl die Leistungselektronik der Kontrolleinheit 80 als auch die Elektronik für die Signalverarbeitung auf die beiden Gehäuseteile 58, 59 verteilt ist.

Für den erfindungsgemässen elektromagnetischen Drehantrieb 1, der die Einmalvorrichtung 100 und die wiederverwendbare Vorrichtung 200 umfasst, ist es ferner ein wichtiger Aspekt 100, dass die Einmalvorrichtung 100, welche vorzugsweise als vormontierte Einheit zusammengesetzt ist, für einige Anwendungen sterilisierbar sein muss. Dabei ist es besonders vorteilhaft, wenn die Einmalvorrichtung 100 gamma-sterilisierbar sind. Bei dieser Art der Sterilisierung wird das zu sterilisierende Element mit Gamma-Strahlung beaufschlagt. Der Vorteil der Gamma-Sterilisierung, beispielsweise im Vergleich zur Dampfsterilisierung, liegt insbesondere darin, dass die Sterilisierung auch durch die Verpackung hindurch erfolgen kann. Gerade bei Einmalteilen ist es eine gängige Praxis, dass die Teile nach ihrer Herstellung in die Verpackung gebracht werden und dann noch eine Zeit lagern, bevor sie an den Kunden ausgeliefert werden. Die Sterilisierung erfolgt dann üblicherweise erst kurz vor der Auslieferung an den Kunden oder erst durch den Kunden. In solchen Fällen erfolgt die Sterilisierung durch die Verpackung hindurch, was bei einer Dampfsterilisierung oder anderen Verfahren nicht möglich ist.

Bezüglich der Einmalteilvorrichtung 100 ist es in der Regel nicht notwendig, dass sie mehr als einmal sterilisierbar sein muss. Dies ist insbesondere bei der Gamma-Sterilisierung ein grosser Vorteil, weil die Beaufschlagung mit Gamma-Strahlung bei Kunststoffen zu Degradationen führen kann, sodass eine mehrfache Gamma-Sterilisierung den Kunststoff unbrauchbar machen kann.

Da in der Regel bei Einmalvorrichtungen 100 auf eine Sterilisierung unter hohen Temperaturen und /oder unter hohem (Dampf-) Druck verzichtet werden kann, können kostengünstigere Kunststoffe eingesetzt werden, beispielsweise solche, die keine hohen Temperaturen aushalten, oder die nicht mehrfach hohen Temperatur- und Druckwerten ausgesetzt werden können.

Unter Berücksichtigung all dieser Aspekte ist es daher bevorzugt, für die Herstellung der Einmalvorrichtung 100 solche Kunststoffe zu verwenden, die zumindest einmal gamma-sterilisierbar sind. Die Materialien sollten dabei gammastabil für eine Dosis von mindestens 40 kGy sein, um eine einmalige Gamma-Sterilisierung zu ermöglichen. Bei der Gamma-Sterilisierung sollten zudem keine giftigen Stoffe entstehen. Zudem ist es bevorzugt, wenn alle Materialien, die mit den zu mischenden bzw. den durchmischten Substanzen in Berührung kommen, USP Class VI Standards erfüllen.

Für die Herstellung der Einmalvorrichtung 100 sind beispielsweise die folgenden Kunststoffe geeignet: PolyVinylChlorid (PVC), PolyPropylene (PP), PolyEthylene (PE), Low Density PolyEthylene (LDPE), Ultra Low Density PolyEthylene (ULDPE), High Density PolyEthylene (HDPE), Ethylene Vinyl Acetate (EVA), PolyEthylene Terephthalate (PET), PolyVinyliDene Fluoride (PVDF), Acrylonitrile Butadiene Styrene (ABS), PolyAcryl, PolyCarbonate (PC), Polysulfone wie beispielsweise Polysulfon (PSU).

Der Verbindungsschlauch 9 bzw. die Verbindungsschläuche 9 sowie zumindest ein Teil der Verbindungen 103 werden vorzugsweise mit Leitungen realisiert, die als flexible Leitungen ausgestaltet sind, also als Leitungen, deren Wandung deformierbar ist. Jede Leitung ist beispielsweise als Kunststoffschlauch ausgestaltet, der beispielsweise aus einem Silikonkautschuk, PVC (Polyvinylchlorid), PU (Polyurethan), PE (Polyethylen), HDPE (High Density Polyethylen), PP (Polypropylen), EVA (Ethyl Vinyl Acetat) oder Nylon besteht.

## Patentansprüche

1. Elektromagnetischer Drehantrieb mit einer Einmalvorrichtung (100), die für den Einmalgebrauch ausgestaltet ist, und mit einer wiederverwendbaren Vorrichtung (200), die für den Mehrfachgebrauch ausgestaltet ist,
wobei die Einmalvorrichtung (100) ein Rotorgehäuse (2) mit einem Rotor (3), einen Verbindungsschlauch (9) für ein Fluid, und mindestens eine weitere Einmalkomponente (10) umfasst, wobei das Rotorgehäuse (2) einen Einlass (21) und einen Auslass (22) aufweist, wobei der Rotor (3) in dem Rotorgehäuse (2) zum Rotieren um eine Solldrehachse vorgesehen ist, welche eine axiale Richtung (A) definiert, wobei der Rotor (3) berührungslos magnetisch antreibbar ist, und wobei der Verbindungsschlauch (9) den Einlass (21) oder den Auslass (22) des Rotorgehäuses (2) mit der weiteren Einmalkomponente (10) verbindet, derart, dass im Betriebszustand das Fluid den Verbindungsschlauch (9) durchströmen kann,
wobei die wiederverwendbare Vorrichtung (200) einen Stator (4) umfasst, mit welchem der Rotor (3) im Betriebszustand berührungslos magnetisch zur Rotation um die Solldrehachse antreibbar ist, wobei der Stator (4) eine Mehrzahl von Wicklungen (6) zur Erzeugung eines elektromagnetischen Drehfelds zum Antreiben des Rotors (3) umfasst, und wobei der Stator (4) ein Statorgehäuse (5) mit einer Durchführung (52) für den Verbindungsschlauch (9) aufweist, **dadurch gekennzeichnet, dass** der Stator (4) öffenbar mit einem ersten Statorteil (48) und einem zweiten Statorteil (49) ausgestaltet ist, welche relativ zueinander bewegbar sind, wobei in einer Offenstellung des Stators (4) der Verbindungsschlauch (9) in die Durchführung (52) einlegbar ist, und wobei in einer Schliessstellung des Stators (4) die Durchführung (52) den Verbindungsschlauch (9) umschliesst.

2. Elektromagnetischer Drehantrieb nach Anspruch 1, wobei der erste Statorteil (48) und der zweite Statorteil (49) mittels eines Gelenks (55) miteinander verbunden sind.

3. Elektromagnetische Drehantrieb nach Anspruch 2, wobei eine elektrische Verbindung (S) vorgesehen ist, welche durch das Gelenk (55) hindurchgeführt ist.

4. Elektromagnetischer Drehantrieb nach einem der vorangehenden Ansprüche, wobei am Statorgehäuse (5) eine mechanische Fixierung (56) vorgesehen ist, mit welcher in der Schliessstellung der erste Statorteil (48) lösbar am zweiten Statorteil (49) fixiert ist.

5. Elektromagnetischer Drehantrieb nach einem der vorangehenden Ansprüche, wobei eine mechanische Zentrierung (571, 572) vorgesehen ist, welches in der Schliessstellung des Stators (4) den ersten Statorteil (48) relativ zu dem zweiten Statorteil (49) zentriert.

6. Elektromagnetischer Drehantrieb nach einem der vorangehenden Ansprüche, wobei das Statorgehäuse (5) im Wesentlichen zylinderförmig ausgestaltet ist, und wobei der erste Statorteil (48) in Umfangsrichtung gesehen länger ausgestaltet ist als der zweite Statorteil (49).

7. Elektromagnetischer Drehantrieb nach einem der vorangehenden Ansprüche, wobei in jedem Statorteil (48, 49) mindestens eine Wicklung (6) vorgesehen ist, und wobei die Anzahl der Wicklungen (6) in dem ersten Statorteil (48) verschieden ist von der Anzahl der Wicklungen (6) in dem zweiten Statorteil (49).

8. Elektromagnetischer Drehantrieb nach einem der vorangehenden Ansprüche, wobei der Stator (4) als Lager- und Antriebsstator ausgestaltet ist, mit welchem der Rotor (3) im Betriebszustand berührungslos magnetisch bezüglich des Stators (4) lagerbar ist.

9. Elektromagnetischer Drehantrieb nach einem der vorangehenden Ansprüche, bei welchem der Rotor (3) einen magnetisch wirksamen Kern (31) umfasst, und bei welchem der Stator (4) eine Mehrzahl von Statorpolen (41) aufweist, die um den magnetisch wirksamen Kern (31) herum angeordnet sind, und von denen jeder jeweils durch eine dem magnetisch wirksamen Kern (31) des Rotors (3) zugewandte Stirnfläche begrenzt wird.

10. Elektromagnetischer Drehantrieb nach Anspruch 9, welcher als Tempelmotor ausgestaltet ist, wobei der Stator (4) eine Mehrzahl von Spulenkernen (45) aufweist, von denen jeder einen stabförmigen Längsschenkel (46) umfasst, welcher sich von einem ersten Ende (461) in axialer Richtung (A) bis zu einem zweiten Ende (462) erstreckt, sowie einen Querschenkel (47), welcher an dem zweiten Ende (462) des Längsschenkels (46) angeordnet ist, und welcher sich in einer radialen Richtung erstreckt, die senkrecht zu der axialen Richtung ist (A), wobei jeder Querschenkel (47) einen der Statorpole bildet (41), und wobei an jedem Längsschenkel (46) mindestens eine konzentrierte Wicklung (6) angeordnet ist, welche den jeweiligen Längsschenkel (46) umgibt.

11. Elektromagnetischer Drehantrieb nach einem der vorangehenden Ansprüche, bei welchem sich der Rotor (3) in axialer Richtung von einem ersten Ende (301) bis zu einem zweiten Ende (302) erstreckt, wobei an jedem Ende (301, 302) ein magnetisch wirksamer Kern (31) vorgesehen ist, und welcher zwei Statoren (4) umfasst, die bezüglich der axialen Richtung (A) beabstandet angeordnet sind, und von denen jeder gemäss einem der vorangehenden Ansprüche ausgestaltet ist, wobei jeder Stator (4) zum Zusammenwirken mit genau einem der magnetisch wirksamen Kerne (31) angeordnet und ausgestaltet ist, derart dass der Rotor (3) im Betriebszustand berührungslos magnetisch bezüglich der Statoren (4) lagerbar ist.

12. Elektromagnetischer Drehantrieb nach einem der vorangehenden Ansprüche, wobei eine Kontrolleinrichtung (80) vorgesehen ist, die an oder in dem ersten oder dem zweiten Statorteil (48, 49) angeordnet ist.

13. Einmalvorrichtung für einen elektromagnetischen Drehantrieb, der nach einem der vorangehenden Ansprüche ausgestaltet ist, welche das Rotorgehäuse (2) mit dem Rotor (3), die Einmalkomponente (10) und den Verbindungsschlauch (9) umfasst.

14. Einmalvorrichtung nach Anspruch 13, bei welcher alle Bestandteile der Einmalvorrichtung zu einer vormontierten Einheit zusammengesetzt sind, welche gesamthaft in die wiederverwendbare Vorrichtung (200) einsetzbar ist.

15. Einmalvorrichtung nach einem der Ansprüche 13-14, welche sterilisiert in einer Verpackung eingeschlossen ist.
